# EUROPÄISCHE PATENTSCHRIFT

(11) **EP 3 303 544 B1**
(45) Veröffentlichungstag und Bekanntmachung des Hinweises auf die Patenterteilung: **02.10.2019**
(21) Anmeldenummer: 16711523.7
(22) Anmeldetag: 08.03.2016
(51) Int. Cl.: C12M 1/00, C12M 3/00, C12M 1/06, C12M 1/34

(54) **BIOREAKTORSYSTEM UND VERFAHREN**
BIOREACTOR SYSTEM AND METHOD
SYSTÈME DE BIORÉACTEUR ET PROCÉDÉ ASSOCIÉ

(30) Priorität: 02.06.2015 DE 102015007060
(43) Veröffentlichungstag der Anmeldung: 11.04.2018
(73) Patentinhaber: Sartorius Stedim Biotech GmbH, 37079 Göttingen (DE)
(72) Erfinder: HUSEMANN, Bernward, 37079 Göttingen (DE); TOPP-MANSKE, Simon, 34253 Lohfelden (DE); NICKEL, Björn, 37235 Hessisch Lichtenau (DE); LEUPOLD, Marco, 37073 Göttingen (DE)
(74) Vertreter: Müller-Boré & Partner Patentanwälte PartG mbB
(86) Internationale Anmeldenummer: PCT/EP2016/000406
(87) Internationale Veröffentlichungsnummer: WO 2016/192823

(56) Entgegenhaltungen:
- WO-A1-2015/039034
- WO-A2-2008/135991
- US-A- 4 541 765
- US-A1- 2003 226 857
- US-A1- 2011 310 696
- US-A1- 2013 081 995

## Beschreibung

Die Erfindung betrifft Bioreaktorsysteme zum Aufnehmen eines Einwegbeutels und ein Verfahren zum Aufnehmen eines Einwegbeutels in ein Bioreaktorsystem.

Bioreaktorsysteme, auch Bioreaktoren genannt, und Pallettanks dienen als Vorrichtung zur Aufnahme und Lagerung von biologischen Medien wie z.B. Fluiden. Biologische Medien können in Einwegbeuteln bereitgestellt werden, die ein Volumen von mehreren hundert Litern umfassen können. Die biologischen Medien werden innerhalb eines Einwegbeutels in den Bioreaktor eingebracht, in dem sie über einen vorbestimmten Zeitraum von üblicherweise mehreren Stunden Dauer auf einer vorbestimmbaren Temperatur temperiert werden. In einem solchen Bioreaktor können unterschiedliche Untersuchungen an dem biologischen Medium vorgenommen werden.

Die Handhabung eines Bioreaktors erfolgt üblicherweise in einer sterilen Umgebung, so dass besonders hohe Anforderungen an die Qualitätssicherung am Bioreaktor gestellt werden. Weiterhin kann die Handhabung der teilweise schweren Einwegbeutel sowie die Bedienung der Bioreaktoren aufwendig und umständlich sein.

Dokument WO 2008/135991 A2 betrifft einen Großbioreaktor mit einer Supportstruktur zur Aufnahme eines Bioreaktors mit mehreren Gaseinlässen und Sammelports. Sämtliche Gaseinlässe und Sammelports werden durch die Supportstruktur geführt.

Dokument US 2013/0081995 A1 betrifft ein Filtersystem für Zellkulturlösungen. Hierbei wird ein Filter verwendet, das eine Einlasskammer von einer Auslasskammer trennt.

Dokument WO 2015/039034 betrifft Bioreaktoren mit mehreren oder verstellbaren Rührern. Hierbei werden zwei oder mehr Rührmotoren verwendet.

Der Erfindung liegt die Aufgabe zugrunde, die Bedienung und/oder Handhabung eines Bioreaktorsystems der eingangs genannten Art zu verbessern.

Diese Aufgabe wird durch die Gegenstände der unabhängigen Ansprüche gelöst. Bevorzugte Ausführungsbeispiele sind die Gegenstände der abhängigen Ansprüche.

Ein erster Aspekt betrifft ein Bioreaktorsystem zum Aufnehmen eines Einwegbeutels mit einem Aufnahmebehälter zum Aufnehmen des Einwegbeutels, wobei eine Bodenfläche des Aufnahmebehälters zum Stützen des Einwegbeutels im Aufnahmebehälter ausgebildet ist. In der Bodenfläche ist eine sich im Bezugssystem der Erde nach unten verjüngende Auslassöffnung zur Aufnahme eines zur Auslassöffnung komplementären Entleerungsanschlusses des Einwegbeutels angeordnet.

Das Bioreaktorsystem kann als ein Bioreaktor, Pallettank und/oder Rührkesselreaktor der eingangs benannten Art ausgebildet sein, der zur Aufnahme von Einwegbeuteln mit einem Volumen von 1 Liter bis zu etwa 2000 Litern ausgebildet sein kann, insbesondere mit einem Volumen von 200 Litern bis 1000 Litern. Das System kann insbesondere zum Aufnehmen von Einwegbeuteln ausgebildet sein, in denen sich ein biologisches Medium wie ein Fluid befindet, das über einen vorbestimmten Zeitraum im System gelagert, temperiert und/oder anders untersucht werden soll.

Der Aufnahmebehälter des Systems stellt einen Behälterinnenraum bereit, der zum Aufnehmen des Einwegbeutels ausgebildet ist. Dabei kann der Aufnahmebehälter zum Aufnehmen eines vorbestimmbaren Typs eines Einwegbeutels ausgebildet sein, also zum Beispiel eines Einwegbeutels eines vorbestimmbaren Herstellers und/oder mit einem vorbestimmbaren Füllvolumen. Insbesondere ist Aufnahmebehälter zur Aufnahme eines Einwegbeutels mit einem Entleerungsanschluss mit einer vorbestimmbaren Form ausgebildet. Behälterwände des Aufnahmebehälters definieren den Behälterinnenraum. Dabei müssen die Behälterwände des Aufnahmebehälters den Behälterinnenraum nicht vollständig umgeben und/oder umgrenzen. So kann der Aufnahmebehälter an seiner Oberkante zum Beispiel eine Rühröffnung aufweisen, durch die eine Rührvorrichtung an den Einwegbeutel im Behälterinnenraum anschließbar ist. Die Rührvorrichtung kann als ein Antrieb ausgebildet sein, der an einen Rührstab angeschlossen werden kann, der im Inneren des Einwegbeutels angeordnet ist. Eine solche Rührvorrichtung ist bevorzugt am oberen Ende des Aufnahmebehälters ausgebildet. Der Aufnahmebehälter kann somit insbesondere deckellos und/oder oben offen ausgebildet sein.

Die Begriffe "oben", "unten", "seitlich", "vertikal", "horizontal", "Höhe" etc. beziehen sich im Rahmen dieser Erfindung auf das Bezugssystem der Erde, in dem das Bioreaktorsystem in einer Betriebsstellung angeordnet ist.

Der Einwegbeutel wird bevorzugt so in den Aufnahmebehälter eingebracht, dass er auf einem Boden des Aufnahmebehälters aufliegt und in direktem, physikalischen Kontakt mit dem Behälterwänden des Aufnahmebehälters steht, insbesondere in Kontakt mit der Bodenfläche des Aufnahmebehälters und ggf. den an die Bodenfläche angrenzenden Behälterwänden. Während das biologische Medium im Aufnahmebehälter untersucht wird, wird der Einwegbeutel durch die Bodenfläche gestützt.

Nach oder während der Untersuchung des biologischen Mediums kann das Medium zumindest teilweise aus dem Aufnahmebehälter ausgelassen werden. Um dazu nicht den ganz schweren Einwegbeutel aus dem Aufnahmebehälter heben zu müssen, wird der Inhalt des Einwegbeutels entleert, während der Einwegbeutel noch im Aufnahmebehälter angeordnet ist. Dazu ist in der Bodenfläche des Aufnahmebehälters die Auslassöffnung ausgebildet, durch die das biologische Medium gezielt abgelassen werden kann. Die Bodenfläche kann dabei zur Auslassöffnung hin trichterförmig ausgebildet sein, wodurch der Einwegbeutel nahezu vollständig entleert werden kann.

Das Bioreaktorsystem ist zur Aufnahme von Einwegbeuteln mit einem sich nach außen, also in Richtung vom Beutelinneren weg, verjüngenden Entleerungsanschluss ausgebildet und vorgesehen. Der Entleerungsanschluss ist im Beutelboden des Einwegbeutels ausgebildet und ragt zumindest teilweise aus dem Einwegbeutel heraus, in Betriebsposition nach unten. Der Entleerungsanschluss ist nicht lediglich in Form eines geraden Zylinders oder einfachen Rohres ausgebildet, sondern weist zumindest teilweise Außenmaße auf, die sich in Richtung vom Beutel weg weisend verjüngen. Genau zu diesem speziellen, sich nach außen hin (in Betriebsposition: nach unten hin) verjüngenden Entleerungsanschluss ist die Auslassöffnung in der Bodenfläche komplementär ausgebildet und umgekehrt.

Wird ein Einwegbeutel mit einem solch komplementären Entleerungsanschluss in das Bioreaktorsystem eingebracht, so rutscht der Entleerungsanschluss im Wesentlichen von alleine und/oder automatisch in die sich nach unten verjüngende Auslassöffnung. Dazu kann die Bodenfläche des Aufnahmebehälters vollständig oder zumindest teilweise in Richtung zur Auslassöffnung hin abfallend ausgebildet sein, um ein Hereinrutschen des komplementären Entleerungsanschlusses in die Auslassöffnung zu unterstützen und/oder zu erleichtern. Die sich nach unten verjüngenden Außenmaße des Entleerungsanschlusses und/oder die sich nach unten verjüngenden Innenflächen der Auslassöffnung wirken dabei als Rutschflächen zum Ausrichten und Anordnen des Entleerungsanschlusses. Zudem kann der Entleerungsanschluss des Einwegbeutels bim Einbringen in den Aufnahmebehälter am Ende eines durch die Auslassöffnung verlegten Schlauches zu der Auslassöffnung hin in seine Sollposition geführt werden.

An dem Entleerungsanschluss des Einwegbeutels können zusätzliche Schläuche und/oder Verschlüsse von Sterilkonnektoren ausgebildet und vorgesehen sein, die ebenfalls durch die Bodenfläche geführt werden können. Die Sterilkonnektoren selbst können größer als der Entleerungsanschluss ausgebildet sein. Die Auslassöffnung der Bodenfläche ist als eine Durchführung ausgebildet, die sowohl die Aufnahme des Entleerungsanschlusses des Einwegbeutels ermöglicht als auch die Durchführung von zumindest einem Schlauch und/oder Verschluss eines Sterilkonnektors. Dabei nimmt die Auslassöffnung den Entleerungsanschluss auf, ohne ihn vollständig durch die Auslassöffnung fallen zu lassen. Der kleinste Innendurchmesser der Auslassöffnung ist deswegen kleiner als der größte Außendurchmesser des komplementären Entleerungsansch lusses.

Durch die vorbestimmte Außenform des Entleerungsanschlusses kann die Auslassöffnung unabhängig von der Größe der ggf. vorhandenen zusätzlichen Sterilkonnektoren genau an die Außenform des komplementären Entleerungsanschlusses angepasst sein. In der Auslassöffnung ist lediglich der komplementäre Entleerungsanschluss gelagert, und ggf. ein (oder mehrere) zusätzlicher Schlauch für die Sterilkonnektoren durchgeführt. Der (oder die) ggf. vorhandene(n) Sterilkonnektor(en) kann (können) unterhalb der Auslassöffnung an das Ende des Schlauchs (der Schläuche) montiert sein. Dadurch können selbst Sterilkonnektoren verwendet werden, die größer als die Auslassöffnung ausgebildet sind.

In Betriebsposition des Bioreaktorsystems ist der komplementäre Entleerungsanschluss des Einwegbeutels in der sich nach unten verjüngenden Auslassöffnung sicher gelagert und/oder gehalten. Der Entleerungsanschluss richtet sich durch die aufeinander abgestimmten Formflächen des Entleerungsanschlusses und der Auslassöffnung selbstständig in der Sollposition aus. Dies gilt auch für die Position einer ggf. am Entleerungsanschluss zentrierten Rührwelle zum Rühren des Inneren des Einwegbeutels. Dies führt zu einem sicheren und verschleißarmen Betrieb der Rührwelle. Zum Auslassen des biologischen Mediums kann der Entleerungsanschluss, z.B. während er in der Auslassöffnung gelagert ist, geöffnet werden.

Diese sichere und effiziente Lagerung des Entleerungsanschlusses in der Auslassöffnung verbessert die Handhabung des Bioreaktorsystems, insbesondere bei der Anordnung des unhandlichen Einwegbeutels im Aufnahmebehälter und beim Auslassen des biologischen Mediums. Gerade am Boden des Aufnahmebehälters ist der Einwegbeutel schwer zugänglich. Die vereinfachte Ausrichtung vereinfacht die Handhabung und insbesondere die Ausrichtung im Aufnahmebehälter deutlich.

Bei einer Ausführungsform ist die Auslassöffnung zumindest teilweise nach unten hin im Wesentlichen konusförmig und/oder im Wesentlichen halbkugelförmig und/oder im Wesentlichen in Form eines Halbellipsoids verjüngend ausgebildet. Die Form eines Konus, einer Halbkugel, eines Halbellipsoids oder z.B. eine Kombination eines Zylinders mit einer dieser Formen eignet sich besonders gut als komplementärer Entleerungsanschluss, der selbsttätig und/oder geführt in die sich in komplementärer Form verjüngende Auslassöffnung rutscht.

Bei einer Ausführungsform weist das Bioreaktorsystem ein Fixierelement auf zum Fixieren des komplementären Entleerungsanschlusses des Einwegbeutels in der Auslassöffnung. Dadurch wird eine Verriegelung der Auslassöffnung bereitgestellt. Das Fixierelement kann als mechanisches Fixierelement wie z.B. als Fixierbolzen ausgebildet sein, der in einen komplementären Eingriff (wie z.B. eine Nut) im Entleerungsanschluss eingreifen kann. Durch das Fixierelement wird der Entleerungsanschluss in Betriebsposition fixiert, was die Ausrichtung und Anordnung des ganzen Einwegbeutels im Aufnahmebehälter verbessert und stabilisiert, insbesondere beim Rühren des biologischen Mediums.

Die Verriegelung und/oder das Fixierelement kann an der Bodenfläche verschraubt, verschweißt und/oder verklebt sein.

Zudem kann das Bioreaktorsystem einen Sensor zum Erfassen des Öffnungszustands der Verriegelung der Auslassöffnung aufweisen.

In einer Weiterbildung weist das Bioreaktorsystem eine Fernbedienung zum Öffnen und/oder Schließen des Fixierelements auf. Die Fernbedienung kann entweder als elektrische Fernbedienung ausgebildet sein oder als mechanische Fernbedienung. Mittels der Fernbedienung kann eine Verriegelung der Auslassöffnung einfach betätigt werden, insbesondere ohne Sichtkontakt zu benötigen zu der ggf. schwer zugänglichen Auslassöffnung. An der Stellung der Fernbedienung kann der Öffnungszustand der Verriegelung erkenntlich sein, ohne die Verriegelung selbst zu sehen. Mittels der Fernbedienung rastet das Fixierelement sicher ein ohne unmittelbare Betätigung des Fixierelements. Dabei wird der Platzbedarf zum Verriegeln an der Bodenfläche reduziert.

Eine mechanische Fernbedienung kann dabei zumindest einen Bedienstab aufweisen, der an die Auslassöffnung heranreicht und mittels dem das Fixierelement verschlossen und/oder geöffnet werden kann. Der Bedienstab kann dabei mechanisch an einen Schließring gekoppelt sein, der um die Auslassöffnung herum angeordnet ist und dessen Drehung mittels des Bedienstabs eine Stellung des Fixierelements steuert. Der Bedienstab kann dabei zumindest einen Meter lang ausgebildet sein, so dass das Fixierelement bequem bedienbar ist. Alternativ kann die Fernbedienung mittels eines Baudenzugs, eines Stellmotors, einer Stange, pneumatisch und/oder hydraulisch implementiert sein.

Bei einer Ausführungsform ist der zur Auslassöffnung komplementäre Entleerungsanschluss als eigenständiges Bauteil des Bioreaktorsystems ausgebildet, das an einem im Einwegbeutel integrierten Standardentleerungsanschluss so befestigbar ist, dass sich der komplementäre Entleerungsanschluss in einer Richtung verjüngt, die vom Beutelinneren des Einwegbeutels weg weist. Der komplementäre Entleerungsanschluss wird vor dem Einbringen des Einwegbeutels in den Aufnahmebehälter am Standardentleerungsanschluss befestigt. Der Standardentleerungsanschluss ist üblicherweise rohrförmig ausgebildet. Um diesen Standardentleerungsanschluss wird der komplementäre Entleerungsanschluss gelegt und/oder an diesem befestigt. Dadurch kann ein beliebiger Einwegbeutel an der Auslassöffnung des Bioreaktors ausgerichtet werden, wobei mit Hilfe des komplementären Entleerungsanschlusses der beliebige Einwegbeutel in einen Einwegbeutel mit sich nach unten verjüngendem Entleerungsanschluss umgewandelt werden kann, der komplementär zur Auslassöffnung ausgebildet ist. Der komplementäre Entleerungsanschluss kann als Einwegbauteil oder als Mehrwegbauteil ausgebildet sein und ist ein Bestandteil des Bioreaktorsystems.

In einer Weiterbildung dieser Ausführungsform ist der komplementäre Entleerungsanschluss als mehrteiliges Bauteil ausgebildet. Der Entleerungsanschluss kann dabei insbesondere zweiteilig ausgebildet sein und zwei Halbschalen aufweisen, die um den Standardentleerungsanschluss gelegt werden können. Die Einzelteile, aus denen sich der Entleerungsanschluss zusammensetzt, können mittels jeweils in die Einzelteile integrierte Fixierelemente relativ zueinander ausgerichtet und/oder fixiert werden. Diese Fixierelemente können Magneten, eine Schnappverriegelung, eine Rastverriegelung und/oder Ausrichtungsstifte mit komplementären Ausrichtungsöffnungen aufweisen.

Ein zweiter Aspekt betrifft ein Bioreaktorsystem zum Aufnehmen eines Einwegbeutels, mit einem Aufnahmebehälter zum Aufnehmen des Einwegbeutels und einer im Wesentlichen an eine Außenwand des Aufnahmebehälters angrenzend angeordneten Arbeitsplattform. Dabei ist eine von einer Bedienperson begehbare Oberfläche der Arbeitsplattform etwa 80 cm bis etwa 140 cm unterhalb einer Oberkante des Aufnahmebehälters angeordnet.

Das Bioreaktorsystem kann als ein Bioreaktor der eingangs benannten Art ausgebildet sein. Das Bioreaktorsystem gemäß dem zweiten Aspekt ist ähnlich zu dem Bioreaktorsystem gemäß dem ersten Aspekt ausgebildet, weswegen viele oder sogar alle Ausführungen zum Bioreaktorsystem gemäß dem ersten Aspekt auch auf das Bioreaktorsystem gemäß dem zweiten Aspekt zutreffen können und umgekehrt.

Beim Bioreaktorsystem gemäß dem zweiten Aspekt ist eine Arbeitsplattform vorgesehen, auf der stehend eine Bedienperson einen komfortablen Zugriff zur Oberkante und somit zur Rühröffnung am Kopfende des Aufnahmebehälters hat. Gerade an der Rühröffnung können weitere Zusatzvorrichtungen wie z.B. ein Filter, zumindest ein Sensor, zumindest eine Sonde, zumindest ein Motor und/oder ähnliche Vorrichtungen angeordnet sein, die die Bedienperson von der Arbeitsplattform aus bedienen kann. Weiterhin können an der Oberkante Leitungen für zusätzlich zugebbare Medien angeordnet sein, deren Zufluss von der Arbeitsplattform aus bequem kontrollierbar und/oder steuerbar ist.

Die Arbeitsplattform ist oberhalb des Bodens ausgebildet, auf dem das Bioreaktorsystem in Betriebsposition angeordnet ist. Die Arbeitsplattform ist im Wesentlichen als eine begehbare Oberfläche ausgebildet, die auf ein Betreten von zumindest einer Person oder bevorzugt von zumindest zwei Personen ausgelegt ist, und somit einer Nutzlast von zumindest 80 kg bzw. zumindest 160 kg standhält. Die Oberfläche ist dabei im Wesentlichen horizontal ausgerichtet.

Die Arbeitsplattform ist angrenzend an eine Außenfläche des Aufnahmebehälters ausgebildet und kann somit bis vollständig an den Aufnahmebehälter heranreichen. Dadurch kann auch die Bedienperson bis an den Aufnahmebehälter herantreten, was eine effiziente und einfache Bedienung der Zusatzvorrichtungen und/oder der Rührvorrichtung an der Oberkante des Aufnahmebehälters ermöglicht.

Die Oberfläche der Arbeitsplattform ist dabei etwa 80 cm bis etwa 140 cm unterhalb der Oberkante des Aufnahmebehälters angeordnet, bevorzugt zwischen etwa 80 cm bis etwa 120 cm, besonders bevorzugt zwischen etwa 95 cm und etwa 110 cm unterhalb der Oberkante des Aufnahmebehälters. Bei dieser Anordnung kann die Oberkante des Aufnahmebehälters bequem von Bedienpersonen unterschiedlicher Körpergröße erreicht werden. Zugleich ist der Abstand derart, dass der Schwerpunkt der meisten Personen unterhalb der Oberkante liegt, weswegen die Bedienperson nicht befürchten müsste, versehentlich in den Aufnahmebehälter zu fallen.

Die Arbeitsplattform ist bevorzugt lediglich an einer Außenfläche des Aufnahmebehälters angeordnet, wie z.B. lediglich an der Rück- oder Vorderseite des Bioreaktorsystems, und nicht umlaufend um den Aufnahmebehälter ausgebildet. Dies spart Platz ein und ist zur Bedienung ausreichend.

Durch die Arbeitsplattform wird einerseits die Verwendung einer zusätzlichen Leiter zum Bedienen des Bioreaktors überflüssig, andererseits kann die Bedienperson auf der Arbeitsplattform bis ganz an den Aufnahmebehälter herantreten und kann dabei sicher auf der Arbeitsplattform stehen. Dadurch wird die Bedienung des Bioreaktors deutlich verbessert und vereinfacht.

Das Bioreaktorsystem gemäß dem zweiten Aspekt kann alle zusätzlichen Merkmale des Bioreaktorsystems gemäß dem ersten Aspekt aufweisen und umgekehrt.

Bei einer Ausführungsform des Bioreaktorsystems mit der Arbeitsplattform weist das Bioreaktorsystem eine integrierte Leiter auf, die von einem Boden, auf dem das Bioreaktorsystem in Betriebsposition angeordnet ist, bis zur begehbaren Oberfläche der Arbeitsplattform führt. Die Leiter vereinfacht die Begehung der Arbeitsplattform und erlaubt den Verzicht auf eine separate Leiter zur Besteigung der Arbeitsplattform. Die Leiter kann in Form von mehreren Trittsprossen ausgebildet sein, die in das Bioreaktorsystem integriert sind.

Bei einer Ausführungsform des Bioreaktorsystems mit der Arbeitsplattform weist die Arbeitsplattform ein Geländer auf, das die Arbeitsplattform wenigstens teilweise umgrenzt. Das Geländer sichert eine Bedienperson auf der Arbeitsplattform in zumindest einer Richtung, z.B. in Richtung von dem Aufnahmebehälter weg. Ein Geländer ist insbesondere bei Arbeitsflächen vorgesehen, die mehr als 65 cm über dem Boden angeordnet sind. Dadurch wird die Bedienung des Bioreaktorsystems sicherer.

In einer alternativen Ausführungsform des Bioreaktorsystems mit der Arbeitsplattform ist die Oberfläche der Arbeitsplattform auf einer Höhe von maximal 65 cm oberhalb des Bodens angeordnet, wobei die Arbeitsplattform geländerlos ausgebildet ist. Auf einer niedrigen Arbeitsplattform kann auf ein Geländer verzichtet werden, ohne die Sicherheit einzuschränken. Dadurch wird die Bewegungsfreiheit und somit der Bedienkomfort erhöht.

Bei einer Ausführungsform des Bioreaktorsystems mit der Arbeitsplattform ist die Arbeitsplattform zumindest teilweise ausziehbar und/oder ausklappbar ausgebildet. Dies ist besonders bei eingeschränktem Platz vorteilhaft, da hier das Bioreaktorsystem sehr kompakt ausgebildet sein kann und besser transportierbar ist.

Eine Ausführungsform betrifft eine Bioreaktorsystem mit einem Aufnahmebehälter zum Aufnehmen eines Einwegbeutels, bei dem unterhalb des Aufnahmebehälters eine Auffangwanne angeordnet zum Auffangen eines aus dem Aufnahmebehälter austretenden Fluids ist. Üblicherweise sollte aus dem Aufnahmebehälter unkontrolliert kein Medium austreten. Bei einem Defekt bzw. Leck im Einwegbeutel und/oder im Aufnahmebehälter sowie bei einem Defekt bzw. Leck im Temperierkreislauf kann die Auffangwanne austretende Flüssigkeit auffangen, bevor diese auf den Boden fließt. Dadurch wird sowohl die Gefahr einer Kontaminierung des Bodens reduziert als auch das Beseitigen der ausgetretenen Flüssigkeit vereinfacht.

In einer Weiterbildung weist die Auffangwanne eine Auskleidung aus Kunststoff auf und/oder ist in ihrer exakten Position unterhalb des Aufnahmebehälters justierbar. Insbesondere kann die Höhe der Auffangwanne über dem Boden einstellbar sein, z.B. zum Ausgleich eines unebenen Bodens. Die Auskleidung der Wanne kann entweder als fest integrierte Kunststoffbeschichtung ausgebildet sein und/oder als einlegbarer Wegwerfbeutel.

Gemäß einer Ausführungsform weist das Bioreaktorsystem zumindest Systemschiene zum Anbringen von zumindest einer Zusatzvorrichtung auf. Die Systemschiene dient zur Befestigung einer Zusatzvorrichtung wie z.B. einer Sonde oder zur Sicherung von Kabeln oder anderen Zuführungen. Mit Hilfe der Systemschiene kann das Bioreaktorsystem individualisiert und auf spezielle Anwendungen abgestimmt werden. Die Systemschiene kann standardisiert sein und/oder zumindest 10 cm lang ausgebildet sein.

Dabei kann das Bioreaktorsystem ein Fenster wie z.B. ein Sicht- und/oder Sondenfenster im Aufnahmebehälter aufweisen, zu dem die Systemschiene benachbart angeordnet ist. Das Fenster ist transparent und dient zur Beobachtung und/oder Kontrolle des biologischen Mediums im Bioreaktor. Bei länglichen Fenstern kann die Systemschiene im Wesentlichen parallel zur Längsachse des Fensters angeordnet sein. Insbesondere kann benachbart zu allen Fenstern zumindest eine Systemschiene an einer Außenwand des Aufnahmebehälters ausgebildet sein.

In einer Weiterbildung ist die Systemschiene in einem Abstand von etwa 3 cm bis etwa 20 cm vom Fenster beabstandet abgeordnet. Bei den Abstandsangaben handelt es sich um einen geringsten Abstand eines Teils der Systemschiene von einem Teil des Fensters. Die Systemschiene kann bevorzugt möglichst nah am Fenster angeordnet sein, um eine Anordnung einer Zusatzvorrichtung möglichst nah am Fenster zu ermöglichen. Die Systemschiene ist nicht weiter als ca. 20 cm, bevorzugt nicht weiter als maximal 10 cm beabstandet vom Fenster angeordnet. So ist die Systemschiene besonders vorteilhaft platziert zum Aufnehmen von zumindest einer Zusatzvorrichtung, die am Fenster angeordnet sein muss, wie z.B. eine Sonde zur Bestimmung von relevanten Prozessparametern im biologischen Medium.

In einer Ausführungsform weist das Bioreaktorsystem zumindest eine zur Oberkante des Aufnahmebehälters benachbarte Systemschiene zum Anbringen von zumindest einer Zusatzvorrichtung auf. Somit wird auch benachbart zur Oberkante, also benachbart zur Rühröffnung des Aufnahmebehälters, die individuelle Anbringung von Zusatzvorrichtungen ermöglicht. Dazu kann die Systemschiene zumindest 10 cm lang ausgebildet sein und/oder in eine im Wesentlichen horizontale Richtung in etwa parallel zur Oberkante des Aufnahmebehälters angeordnet sein.

Ein dritter Aspekt betrifft ein Bioreaktorsystem zum Aufnehmen eines Einwegbeutels, mit einem Aufnahmebehälter zum Aufnehmen des Einwegbeutels und einer Kabelführung an einer Außenwand des Aufnahmebehälters, wobei die Kabelführung bis zu einer Oberkante des Aufnahmebehälters ausgebildet ist. Die Kabelführung kann auch als Schlauchführung oder als kombinierte Kabel- und Schlauchführung ausgebildet sein.

Das Bioreaktorsystem kann als ein Bioreaktor der eingangs benannten Art ausgebildet sein. Das Bioreaktorsystem gemäß dem dritten Aspekt ist ähnlich zu dem Bioreaktorsystem gemäß dem ersten und/oder zweiten Aspekt ausgebildet, weswegen viele oder sogar alle Ausführungen zum Bioreaktorsystem gemäß dem ersten und/oder zweiten Aspekt auch auf das Bioreaktorsystem gemäß dem dritten Aspekt zutreffen können und umgekehrt.

Beim Bioreaktorsystem gemäß dem dritten Aspekt eine Kabelführung an einer Außenwand des Aufnahmebehälters vorgesehen, die in etwa bis zur Oberkante des Aufnahmebehälters ausgebildet ist. Die Kabelführung kann im Wesentlich U-förmig ausgebildet sein, wobei Kabel und/oder Schläuche in die U-Öffnung eingebracht und dort sicher geführt werden. Die Kabelführung kann zusätzlich oder alternativ zumindest teilweise als ein Gitterkabelkanal ausgebildet sein. Allgemein ist die Kabelführung so ausgebildet, dass sie Kabel und/oder Schläuche sicher aufnimmt. Damit sind die Kabel und/oder Schläuche in einer festen Sollposition am Aufnahmebehälter gesichert und geführt, wodurch die Wahrscheinlichkeit einer Störung einer Bedienperson durch die Kabel und/oder Schläuche reduziert wird, ebenso wie die Gefahr einer versehentlichen Entkopplung eines Kabels und/oder Schlauchs und/oder einer Zuleitung. Die Kabelführung reicht dabei bis zur Oberkante des Aufnahmebehälters und somit bis zur Rühröffnung. Gerade bis zur Rühröffnung müssen z.B. Stromkabel für einen Rührmotor und/oder weitere Zuleitungen für Sensoren verlegt sein, die von oben in die Rühröffnung herein reichen. Zudem können an der Rühröffnung weitere Zusatzmedienbehälter angeordnet sein, über die zusätzliche Medien in den Aufnahmebehälter zugeleitet werden können. Hierbei können Zuleitungen bzw. Schläuche zu den Zusatzmedienbehältern durch die Kabelführungen geführt sein.

Durch die Kabelführung wird eine Stolpergefahr reduziert. Durch die Kabelführung wird außerdem die Gefahr einer Beschädigung der Schläuche und Kabel verringert. Dadurch wird die Bedienung des Bioreaktors benutzerfreundlicher.

Das Bioreaktorsystem gemäß dem dritten Aspekt kann alle zusätzlichen Merkmale des Bioreaktorsystems gemäß dem ersten und/oder zweiten Aspekt aufweisen und umgekehrt.

Gemäß einer Ausführungsform des Bioreaktors mit der Kabelführung ist die Kabelführung zumindest teilweise mäanderförmig ausgebildet ist. Insbesondere kann die Kabelführung Steckklappen aufweisen, die mäanderartig vor eine Führungsrinne der Kabelführung greifen. Die Kabel und/oder Leitungen können dabei schnell und sicher hinter die mäanderartigen Steckklappen gesteckt bzw. angeordnet werden und sind dort geführt.

Gemäß einer Ausführungsform des Bioreaktors mit der Kabelführung weist das Bioreaktorsystem einer Trägerbrücke auf, die auf der Oberkante des Aufnahmebehälters angeordnet ist. Dabei ist die Trägerbrücke zumindest an einer Stelle der Oberkante des Aufnahmebehälter angeordnet ist, zu der die Kabelführung führt. Auf der Trägerbrücke kann/können eine Rührvorrichtung und/oder Sensoren des Bioreaktorsystems angeordnet sein. Die Kabelführung führt die Kabel und/oder Leitungen direkt an zumindest einen Ansatzpunkt der Trägerbrücke, von wo das Kabel bzw. die Leitungen weitergeführt werden auf die Trägerbrücke und zu den dort angeordneten Vorrichtungen. Bevorzugt weist das Bioreaktorsystem zwei solche Kabelführungen auf, die im Wesentlichen symmetrisch zueinander ausgebildet sind und die zu den zwei Ansatzpunkten der Trägerbrücke auf der Oberkante des Aufnahmebehälters führen.

In einer Weiterbildung dieser Ausführungsform verläuft die Kabelführung von der Oberkante des Aufnahmebehälters an dessen Außenwand entlang im Wesentlichen vertikal nach unten bis zu einem Umschwenkpunkt, an welchem die Kabelführung von der Außenwand des Aufnahmebehälters weg zu einem Außenbereich des Bioreaktorsystems verläuft. Somit verläuft die Kabelführung zumindest teilweise im Wesentlichen vertikal, und zwar bis zur und/oder weg von der Oberkante des Aufnahmebehälters, also in einem oberen Bereich des Biorekatorsystems. Damit ist die Kabelführung zumindest in diesem oberen Bereich sehr Platz sparend ausgebildet und erleichtert damit die Bedienung bzw. den Zugriff auf die Rühröffnung des Bioreaktorsystems. Von der Oberkante aus führt die Kabelführung frühestens am Umschwenkpunkt von der Außenwand des Aufnahmebehälters weg, und somit an einem Bereich, der z.B. unterhalb der Arbeitsplattform angeordnet ist und den Zugriff auf den Bioreaktor kaum stört.

In einer Weiterbildung der Ausführungsform mit der Kabelführung ist diese zweiteilig ausgebildet mit zumindest einer Permanentkabelführung und zumindest einer Zusatzkabelführung. Die Trennung in Permanent- und Zusatzkabelführung ermöglicht einerseits ein fixiertes und sicheres Führen der permanent verlegten Kabel und/oder Leitungen, andererseits ein komfortables und sicheres Verlegen von zusätzlichen Kabeln und/oder Leitungen, die anwendungsspezifisch sind und nicht immer benötigt werden.

Gemäß einer Ausführungsform weist das Bioreaktorsystem eine Verdunklungsvorrichtung für zumindest ein Sichtfenster und/oder eine Verdunklungsabdeckung zum Verdunkeln einer Öffnung an einer Oberkante des Aufnahmebehälters auf. Insbesondere kann für jedes Sichtfenster im Aufnahmebehälter eine Verdunklungsvorrichtung vorgesehen sein, z.B. in Form eines Rollos, Schiebers, eine elektrochrome Scheibe aus einem elektrisch verdunkelbarem Material, etc. Durch die Verdunklungsvorrichtung werden gleichmäßige Reaktionsbedingungen ermöglicht und insbesondere die Gefahr einer Beeinflussung der biologischen Reaktion durch Licht reduziert. Gleiches gilt für die Verdunklungsabdeckung, die z.B. in Form einer Plane ausgebildet sein kann und über die Rühröffnung gelegt werden kann.

Ein vierter Aspekt betrifft ein Verfahren zum Aufnehmen eines Einwegbeutels in ein Bioreaktorsystem, mit den Schritten:
- Bereitstellen eines Bioreaktorsystems mit einem Aufnahmebehälter, in dessen Bodenfläche eine sich im Bezugssystem der Erde nach unten verjüngende Auslassöffnung ausgebildet ist;
- Bereitstellen eines Einwegbeutels mit einem Standardentleerungsanschluss;
- Anbringen eines zur Auslassöffnung komplementären Entleerungsanschlusses an den Standardentleerungsanschluss des Einwegbeutels;
- Einbringen des Einwegbeutels in den Aufnahmebehälter des Bioreaktorsystems, wobei der komplementäre Entleerungsanschluss in der Auslassöffnung angeordnet wird.

Das Bioreaktorsystem kann dabei insbesondere als ein Bioreaktorsystem gemäß dem ersten Aspekt ausgebildet sein. Der komplementäre Entleerungsanschluss ist als separates Bauteil ausgebildet, dass an und/oder um den Standardentleerungsanschluss des Einwegbeutels herum angeordnet wird.

In einer Weiterbildung wird ein Schlauch durch die Auslassöffnung geführt, dessen eines Schlauchende am komplementären Entleerungsanschluss angebracht wird. Hierbei wird der komplementäre Entleerungsanschluss mit Hilfe des Schlauchs in die sich nach unten verjüngende Auslassöffnung geführt, z.B. indem der Schlauch durch die Auslassöffnung nach unten gezogen wird und mit ihm der komplementäre Entleerungsanschluss in die Auslassöffnung.

Durch das Verfahren gemäß dem vierten Aspekt wird die Ausrichtung und das Einbringen eines beliebigen Einwegbeutels mit einem beliebigen Standardentleerungsanschluss in das Bioreaktorsystem vereinfacht und verbessert.

Die Erfindung wird nachfolgend anhand von in Figuren gezeigten Ausführungsformen näher beschrieben. Einzelne Merkmale der in den Figuren gezeigten Ausführungsformen können in anderen Ausführungsformen realisiert werden. Einige gleiche oder ähnliche Merkmale der Ausführungsformen sind dabei mit den gleichen Bezugszeichen bezeichnet. Es zeigt:
- Figur 1: in einer perspektivischen Darstellung eine Vorrichtung zum Aufnehmen eines Einwegbeutels;
- Figur 2A: in einer ersten Seitenansicht eine Vorrichtung zum Aufnehmen eines Einwegbeutels;
- Figur 2B: in einer zweiten Seitenansicht eine Vorrichtung zum Aufnehmen eines Einwegbeutels;
- Figur 3: in einer perspektivischen Darstellung eine vertikale Schnittansicht durch eine Vorrichtung zum Aufnehmen eines Einwegbeutels;
- Figur 4A: in einer Seitenansicht eine Vorrichtung zum Aufnehmen eines Einwegbeutels mit einer hohen Arbeitsplattform;
- Figur 4B: in einer Seitenansicht eine Vorrichtung zum Aufnehmen eines Einwegbeutels mit einer niedrigen Arbeitsplattform;
- Figur 5: in einer perspektivischen Darstellung einen zweiteiligen konischen Entleerungsanschluss;
- Figur 6A: in einer Draufsicht den in Figur 5 gezeigten zweiteiligen konischen Entleerungsanschluss;
- Figur 6B: einen Querschnitt durch den in Figur 5 gezeigten zweiteiligen konischen Entleerungsanschluss;
- Figur 6C: einen Querschnitt durch den in Figur 5 gezeigten zweiteiligen konischen Entleerungsanschluss in einer Betriebsposition, in der er um einen Standardentleerungsanschluss gelegt ist;
- Figur 7A: in einer Bodenansicht eine sich nach unten verjüngenden Auslassöffnung mit geöffnetem Fixierelement sowie zugehöriger mechanischer Fernbedienung;
- Figur 7B: in einer Bodenansicht eine sich nach unten verjüngenden Auslassöffnung mit geschlossenem Fixierelement sowie zugehöriger mechanischer Fernbedienung;
- Figur 7C: in einer Seitenansicht eine sich nach unten verjüngenden Auslassöffnung mit zugehöriger mechanischer Fernbedienung;
- Figur 8A: in einer Seitenansicht eine Vorrichtung zum Aufnehmen eines Einwegbeutels mit einer Arbeitsplattform; und
- Figur 8B: in einer perspektivischen Darstellung eine Vorrichtung zum Aufnehmen eines Einwegbeutels mit einer Arbeitsplattform.

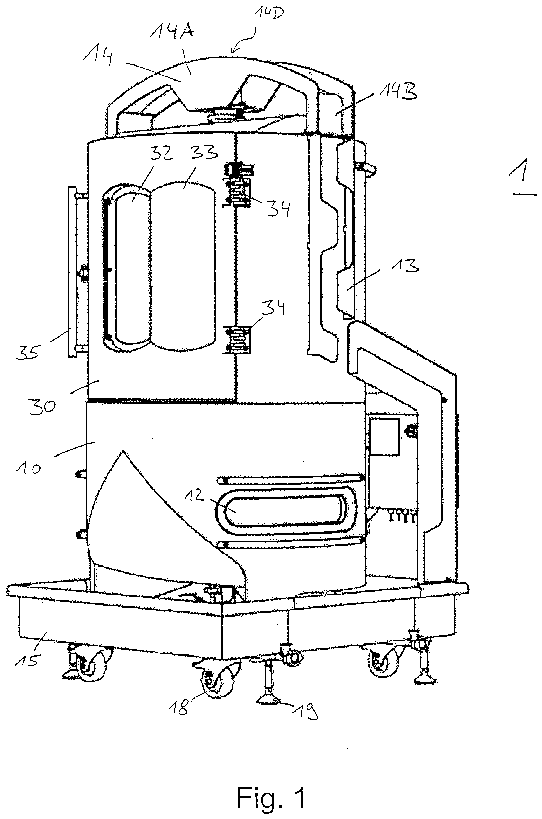

**Figur 1** zeigt in einer perspektivischen Darstellung eine Vorrichtung 1 zum Aufnehmen eines Einwegbeutels. Die in den Figuren gezeigte Vorrichtung 1 kann als ein Bestandteil eines Systems und/oder als Bioreaktorsystem zum Aufnehmen eines Einwegbeutels ausgebildet sein.

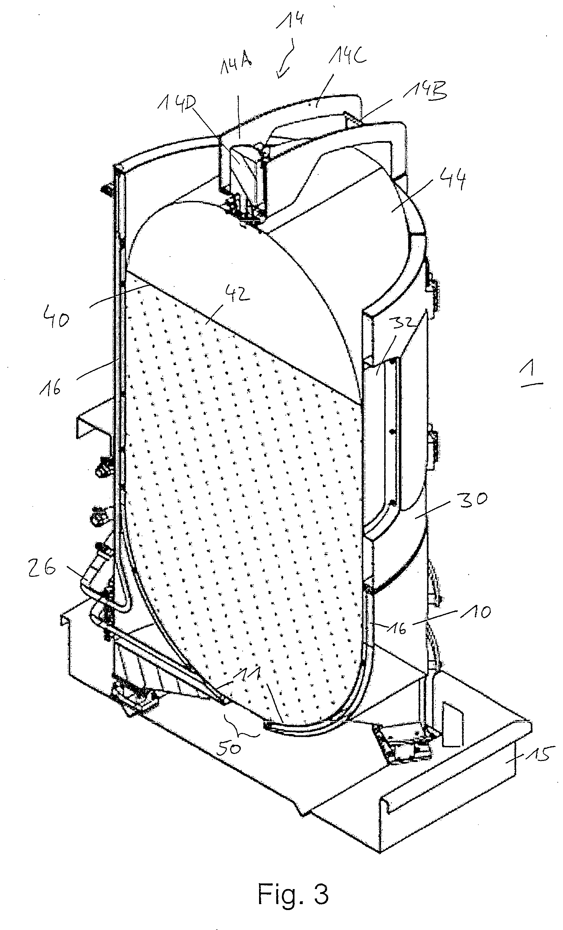

Die Vorrichtung 1 weist einen Aufnahmebehälter 10 auf, der im Wesentlichen die Form eines vertikal angeordneten Zylinders aufweist, d.h. dessen Zylinderachse im Wesentlichen vertikal angeordnet ist. Der Aufnahmebehälter 10 weist einen Behälterinnenraum auf, in den ein Einwegbeutel 44 (siehe **Figur 3**) zumindest teilweise eingebracht werden kann, der zum Beispiel ein biologisches Medium beinhalten kann. Das biologische Medium im Einwegbeutel 44 wird im Behälterinnenraum des Aufnahmebehälters 10 über einen spezifischen (vorbestimmten bzw. vorbestimmbaren) Zeitraum gelagert. Während sich der Einwegbeutel 44 mit dem biologischen Medium im Inneren des Aufnahmebehälters 10 befindet, können sich unterschiedliche Reaktionen mit oder an dem biologischen Medium ereignen. Somit kann die Vorrichtung 1 auch als Bioreaktor und/oder Bioreaktorsystem ausgebildet sein.

Zur Beobachtung des biologischen Mediums sind in der Seitenwand bzw. den Seitenwänden ein oder mehrere, zum Teil unterschiedliche Sichtfenster 12, 32 ausgebildet, durch das bzw. die von außen durch die Behälterwand hindurch in den Behälterinnenraum des Aufnahmebehälters 10 hineingeblickt werden kann, um das biologische Medium zu beobachten. Die Vorrichtung 1 weist dazu im unteren Drittel zwei Bodenfenster 12 auf, sowie ein Türsichtfenster 32. Die Bodenfenster 12 können zum Einstecken zumindest einer Sonde und/oder zumindest eines Schlauchs in den Einwegbeutel dienen. sind bevorzugt im Wesentlichen in Form eines länglichen Ovals ausgebildet, dessen lange Ovalachse im Wesentlichen horizontal entlang der gekrümmten Zylinderaußenwand des Aufnahmebehälters 10 ausgerichtet ist. Das Türsichtfenster 32 ist bevorzugt im Wesentlichen in Form eines länglichen Rechtecks ausgebildet, wobei deren längere Seiten im Wesentlichen vertikal ausgerichtet sind. Das Türsichtfenster 32 ist bevorzugt etwa in der Mitte einer Einblatttür 30 in der Behälterwand des Aufnahmebehälters 10 ausgebildet.

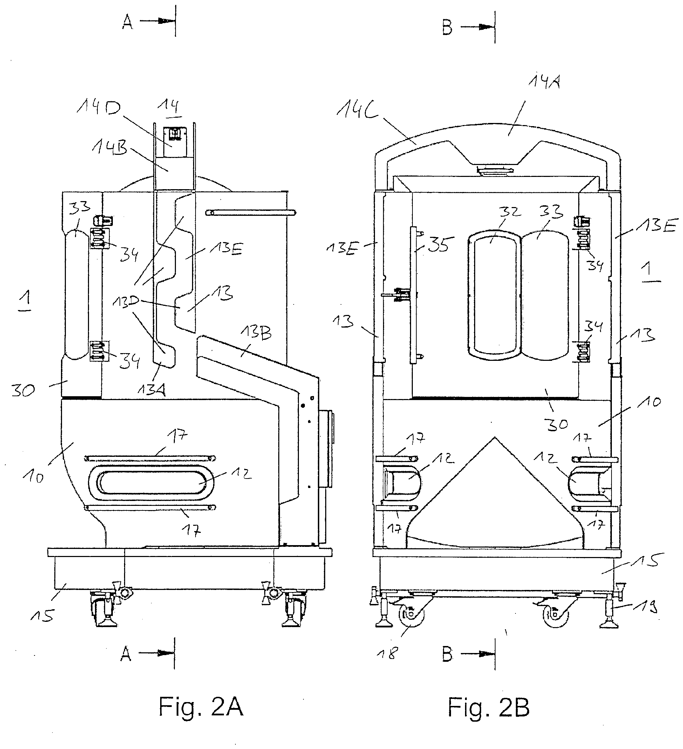

**Figuren 2A und 2B** zeigen zusammen mit Figur 1 unterschiedliche Ansichten der Vorrichtung 1. So zeigt z.B. Figur 2B eine frontale Seitenansicht auf die Einblatttür 30. Die Einblatttür 30 erstreckt sich in der Breite, also in horizontaler Richtung, in etwa über ein Mantelflächen- bzw. Zylindersegment des Aufnahmebehälters 10 von etwa 70° bis etwa 160° (z.B. von ca. 100°). In horizontaler Richtung erstreckt sich die Einblatttür 30 bevorzugt von zwei Türscharnieren 34 entlang des Zylindermantels bis zu einem Türgriff 35 am gegenüberliegenden Türende. Die Einblatttür 30 ist bevorzugt im Wesentlichen in der oberen Hälfte, besonders bevorzugt in den oberen zwei Dritteln des Aufnahmebehälters 10 ausgebildet, während die untere Hälfte (bevorzugt das untere Drittel) des Aufnahmebehälters 10 bevorzugt im Wesentlichen in Form einer steifen Bodenschale ausgebildet ist, die selber nicht öffenbar ausgebildet ist. Die Einblatttür 30 ist um die Türscharniere 34 drehbar bzw. schwenkbar und somit öffenbar. Ist die Einblatttür 30 geöffnet, so ist in dem Aufnahmebehälter 10 an einer seitlichen Position eine Türöffnung ausgebildet, durch die ein Zugriff auf den Behälterinnenraum des Aufnahmebehälters 10 ermöglicht wird. Durch die Türöffnung kann zum Beispiel der Einwegbeutel 44 bevorzugt von einer im Wesentlichen seitlichen bzw. radialen Richtung her, also im Wesentlichen in einer horizontalen Bewegungsrichtung, in den Behälterinnenraum des Aufnahmebehälters 10 eingeführt werden.

An dem Türsichtfenster 32 ist bevorzugt eine Verdunklungsvorrichtung in Form einer Türverdunklung 33 angeordnet, die vor das Türsichtfenster 32 geklappt, geschoben, gerollt werden kann oder anders zur Verdunklung des Türsichtfensters 32 dient. Die Türverdunklung 33 dient dem Verdunkeln des Behälterinnenraums. Eine ähnliche oder anders ausgebildete (Boden-)Verdunklung kann bevorzugt auch für die Bodenfenster 12 und gegebenenfalls weitere Sichtfenster vorgesehen sein.

Die Vorrichtung 1 ist auf Rollen 18 rollbar bzw. verlagerbar gelagert, auf denen die Vorrichtung z.B. durch einen Raum geschoben werden kann. Zusätzlich zu den Rollen 18 kann die Vorrichtung 1 am unteren Ende Fixierfüße 19 aufweisen, die zum Fixieren und zum Einstellen der Höhe der Vorrichtung 1 dienen. Die Fixierfüße 19 können auch zum Ausgleich eines unebenen Bodens verwendet werden.

Unterhalb des Aufnahmebehälters 10 ist eine Auffangwanne 15 angeordnet, die nach oben geöffnet ist und zum zumindest teilweisen Auffangen von einem gegebenenfalls aus dem Behälterinnenraum austretenden biologischen Medium und/oder einem Temperiermedium dient. Die Auffangwanne 15 kann bevorzugt eine Auskleidung aus Kunststoff aufweisen, die an die Dimension der Auffangwanne 15 angepasst ist. Dadurch wird die gesamte Vorrichtung 1 geschützt, insbesondere ggf. vorhandene Gewichtszellen und/oder Wägezellen. Weiterhin wird durch die Auskleidung die Biosicherheit verbessert, z.B. durch einfache Entfernung von kontaminerendem Biomaterial aus der Auffangwanne 15 anstatt anschließender Reinigung derselben.

Die Auffangwanne 15 ist durch eine Verstelleinrichtung verstellbar. Insbesondere kann die relative Höhe der Auffangwanne 15 mittels der Verstelleinrichtung justiert werden und/oder die Ausrichtung unterhalb des Aufnahmebehälters 10.

Benachbart zu den Bodenfenstern 12 und/oder zum Türsichtfenster 32 können ein oder mehrere Systemschienen 17 an der Vorrichtung 1 angeordnet sein, an die ein oder mehrere Zusatzgeräte (wie zum Beispiel Sonden und/oder weitere Halterungen für Spezialgeräte wie Probeentnahmesysteme, Schläuche oder andere so mechanisch entlastbare Verbindungen) mit dem Einwegbeutel verbindbar und/oder befestigbar sind. Bei der Vorrichtung 1 sind ein oder mehrere im Wesentlichen horizontal verlaufende Systemschienen 17 benachbart zu den Bodenfenstern 12 angeordnet, und zwar bevorzugt jeweils eine Systemschiene 17 oberhalb und unterhalb jedes Bodenfensters 12.

Der Aufnahmebehälter 10 ist im Wesentlichen nach oben offen ausgebildet. An Stelle eines Zylinderdeckels bzw. unabhängig hiervon weist der Aufnahmebehälter 10 eine Rühröffnung auf, die bevorzugt an oder Nahe der Oberkante des Aufnahmebehälters 10 ausgebildet ist. Oberhalb des nach oben offenen Aufnahmebehälters 10 ist eine Rührvorrichtung 14 ausgebildet bzw. angeordnet, über die ein Rührstab durch die Rühröffnung hindurch mit dem Einwegbeutel 44 so verbunden werden kann, dass das Innere des Einwegbeutels 44 durchmischt werden kann. Der Rührstab kann im Inneren des Einwegbeutels angeordnet sein und über eine Kopplung bzw. Kupplung mit der Rührvorrichtung 14 verbunden werden. Die Rührvorrichtung 14 ist bevorzugt zentral über dem Aufnahmebehälter 10 ausgebildet und wird von einer Trägerbrücke 14A-C getragen, die auf bzw. an einem oberen Rand des Aufnahmebehälters 10 an einander im Wesentlichen gegenüberliegenden Seitenwänden des Aufnahmebehälters 10 an- bzw. aufliegt. Die Trägerbrücke weist ein oder mehrere Trägerelemente 14C auf, die sich von zumindest einer Seitenwand des Aufnahmebehälters 10 zumindest teilweise über die obere Öffnung hiervon erstreckt. Insbesondere sind zwei Trägerelemente 14C vorgesehen, die sich quer über den Aufnahmebehälter 10 erstrecken und sich auf in etwa gegenüberliegenden Bereichen auf dem Aufnahmebehälter 10 abstützen bzw. von diesem getragen werden. Die zwei Trägerelemente 14C sind durch ein oder mehrere (z.B. zwei) Querelemente 14B miteinander verbunden. In einem mittleren Bereich der Trägerbrücke ist eine Rührelementaufnahme 14A vorgesehen, an der Rührelemente der Rührvorrichtung anbringbar sind, um über den Rührstab durch die Rühröffnung des Einwegbeutels 44 dessen Inhalt zu durchmischen bzw. rühren. Bevorzugt ist ein Motor 14D an der Rührelementaufnahme 14A befestigt, der den Rührstab antreibt. Die zwei Trägerelemente 14B und/oder die Rührelementaufnahme 14A sind derartig wangenartig ausgebildet, dass sie den Motor 14D seitlich schützen, so dass dieser vor z.B. unbeabsichtigter Kollision mit externen Objekten (z.B. einen Tragekran) geschützt ist. Ferner kann die Rührelementaufnahme 14A und/oder ein oder mehrere Trägerelemente 14C einen Abstützbereich 14E oberhalb des Aufnahmebehälters 10 bzw. des Einwegbeutels 44 aufweisen, an dem ein oder mehrere zusätzliche Prozesseinrichtungen (z.B. Filter, Sicherheitsventil(e) und/oder Sensor(en)) anordenbar sind, mittels derer der Inhalt des Einwegbeutels 44 prozessiert und/oder analysiert und/oder in einem spezifischen (vorbestimmten bzw. vorbestimmbaren) Zustand (z.B. Temperatur, Druck, CO₂-Gehalt und/oder O₂-Gehalt) gehalten bzw. versetzt werden kann. An den Ansatzpunkten der Trägerbrücke an den Behälterwänden (insbesondere an dem Übergang zwischen Trägerelement(2) 14C und Seitenwand/wände des Aufnahmebehälters 10) ist zumindest eine Kabelführung 13 ausgebildet bzw. schließt diese an.

Figur 2A zeigt eine Seitenansicht auf eine der beiden Kabelführungen 13. Die zweite der beiden Kabelführungen 13 ist an der gegenüberliegenden Außenwand des Aufnahmebehälters 10 angeordnet. Die Seitenansicht der Figur 2A zeigt die Vorrichtung 1 in einer Position, die gegenüber der Seitenansicht der Figur 2B um 90° gedreht ist.

Die Kabelführung 13 weist eine Oberkabelführung 13E auf, die im Wesentlichen von der Oberkante des Aufnahmebehälters 10 vertikal nach unten verläuft, und zwar zumindest bis zur Hälfte der Höhe des Aufnahmebehälters 10, nämlich bis zu einem Umschwenkpunkt 13A. Vom Umschwenkpunkt 13A an verläuft die Kabelführung 13 weiter als Unterkabelführung 13B seitlich und/oder nach hinten weg. Der vertikale Teil der Kabelführung 13 (d.h. die Oberkabelführung 13E) weist ein oder mehrere Steckklappen 13D auf, hinter die (bzw. außerhalb derer) Kabel und/oder Leitungen so gesteckt bzw. angeordnet werden können, dass die Kabel und/oder Leitungen sowohl von der Außenwand des Aufnahmebehälters 10 nach innen hin, als auch nach außen hin durch die Steckklappe(n) 13D geführt und/oder sicher gehalten sind. Die Steckklappen 13E greifen dabei mäanderartig (bzw. seitlich abwechselnd) vor die Oberkabelführung 13E, so dass die Kabelführung 13 bevorzugt zumindest teilweise im Wesentlichen mäanderförmig ausgebildet ist.

Die Kabelführung 13 kann Formbauteile aufweisen, in die die ein oder mehrere Kabel und/oder ein oder mehrere Schläuche und/oder ein oder mehrere Leitungen angeordnet bzw. eingelegt werden können. Weiterhin kann die Kabelführung 13 bevorzugt zumindest eine Zugentlastung aufweisen. Die Kabelführung 13 reduziert die Gefahr einer ungewollten Wechselwirkung eines Benutzers (z.B. eine Stolpergefahr) und erhöht somit die Sicherheit bei verbesserter Handhabung.

**Figur 3** zeigt in einer perspektivischen Darstellung eine Ansicht eines vertikalen Schnitts durch die Vorrichtung 1. In Figur 3 gezeigt z.B. ist ein Einwegbeutel 44, genauer ein Schnitt durch diesen Einwegbeutel 44, der im Behälterinnenraum des Aufnahmebehälters 10 angeordnet ist. Im Behälterinnenraum des Aufnahmebehälters 10 und zugleich auch im Inneren des Einwegbeutels 44 ist ein biologisches Medium 42 angeordnet, das bis auf Höhe einer vorbestimmten Füllstandhöhe 40 gefüllt ist. Das biologische Medium 42 erstreckt sich vom Boden des Aufnahmebehälters 10 bis hin zu einer Füllstandhöhe 40 und füllt somit bevorzugt das gesamte Innenvolumen des Aufnahmebehälters 10 bis hin zur Füllstandhöhe 40, abzüglich des Volumens der Wände des Einwegbeutels 44, die jedoch sehr dünn ausgebildet sind und kaum ins Gewicht fallen.

Der Einwegbeutel 44 wird durch eine Behälterwand 16 des Aufnahmebehälters 10 gestützt bzw. im Wesentlichen in Form gehalten, die sich bevorzugt von einer abgerundeten Bodenfläche 11 des Aufnahmebehälters 10 bis über die Füllstandhöhe 40 hinaus nach oben erstrecken. Somit wird kann die Bodenfläche 11 zum Stützen des Einwegbeutels 44 ausgebildet sein. Zumindest entlang der oberen Hälfte, bevorzugt entlang der oberen zwei Drittel des Aufnahmebehälters 10, kann sich die Behälterwand 16 im Wesentlichen in vertikaler Richtung senkrecht nach oben erstrecken.

An den Innenwänden des Aufnahmebehälters 10 ist eine Temperierwand bzw. Temperierhohlwand 20 angeordnet, in der ein (in den Figuren nicht gezeigtes) Temperiermedium angeordnet ist bzw. werden kann. Das Temperiermedium wird bevorzugt auf einen Niedrigdruck kleiner als 1 bar oder kleiner als 0,5 bar geregelt. Die Temperierhohlwand 20 erstreckt sich über den gesamten Boden des Aufnahmebehälters 10 und entlang der Behälterwände von der Bodenfläche 11 an nach oben bis über die Füllstandhöhe 40 hinaus.

Das biologische Medium 42 steht (bevorzugt in direktem Wärmekontakt) zu der Temperierhohlwand 20 bzw. ist mit diesem in Wärmekontakt bringbar, von der es bevorzugt lediglich über die dünne Beutelwand des Einwegbeutels 44 getrennt ist. Über das Temperiermedium kann das biologische Medium auf eine vorbestimmbare Temperatur geregelt werden.

Die Vorrichtung 1 kann insbesondere dazu ausgebildet und vorgesehen sein, den Behälterinnenraum auf eine spezifische (vorbestimmte bzw. vorbestimmbare) Temperatur zu temperieren.

Die Temperierhohlwand 20 ist an ihrer Außenseite zumindest teilweise von einer Isolierhohlwand 25 umgeben, in der sich bevorzugt eine Isolierung (z.B. PU Schaum oder Vakuum) befindet. Die Isolierhohlwand 25 umgibt den Behälterinnenraum des Aufnahmebehälters 10 bevorzugt beinahe vollständig vom Boden des Aufnahmebehälters 10 bis hin zum oberen Ende der Behälterwand 16 (vgl. dazu auch Figur 3). Die Isolierhohlwand 25 isoliert sowohl den Behälterinnenraum, als auch insbesondere die Temperierhohlwand 20 nach außen hin. Durch die in der Isolierhohlwand 25 angeordnete Isolierung wird eine gerichtete Temperierung mittels des Temperiermediums nach innen hin zum Behälterinnenraum bereitgestellt, was die Energieeffizienz der Vorrichtung 1 erhöht.

Das Temperiermedium befindet sich bevorzugt im Inneren eines abgeschlossenen Temperiersystems, das das Innere der Temperierhohlwand 20 umfasst. Die Regelung und/oder Steuerung der Temperatur des Temperiermediums kann über eine interne, elektrisch betriebene Heizvorrichtung erfolgen sowie optional oder alternativ über einen externen Wärmetauscher. Über den externen Wärmetauscher kann sowohl eine Kühlung als auch eine Erwärmung des Temperiermediums unabhängig von der internen elektrischen Heizvorrichtung des Temperiersystems erfolgen.

Der Einwegbeutel 44 wird z.B. nach Beenden der biologischen Reaktion durch eine unterhalb der Vorrichtung 1 angeordnete Auslassöffnung 5 zumindest teilweise entleert und kann anschließend vollständig entsorgt werden. Dabei ist ein Entleerungsanschluss 45 des Einwegbeutels 44 in der Auslassöffnung 5 gelagert bzw. vorgesehen, über den der Einwegbeutel 44 entleerbar ist. Die Auslassöffnung 5 ist in der Bodenfläche 11 des Aufnahmebehälters 10 ausgebildet. Durch Verwendung des Einwegbeutels 44 kann eine Reinigung der Vorrichtung 1 eingespart werden, bzw. wesentlich schneller erfolgen.

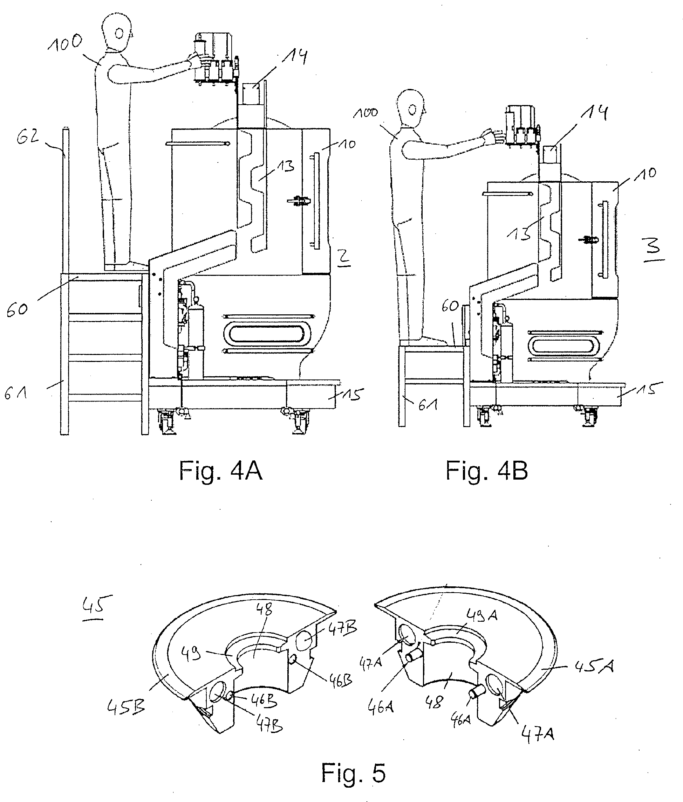

**Figur 4A** zeigt in einer Seitenansicht eine Vorrichtung 2 zum (zumindest teilweisen) Aufnehmen eines Einwegbeutels mit einer (insbesondere hohen bzw. erhöhten) Arbeitsplattform 60. Die Vorrichtung 2 kann insbesondere als Bioreaktor und/oder Bioreaktorsystem zum Aufnehmen eines Einwegbeutels ausgebildet sein.

An einer Seite des Bioreaktors 2 ist die Arbeitsplattform 60 ausgebildet, auf der eine Bedienperson 100 steht und die Rührvorrichtung 14 bedient. Weiterhin hat die Bedienperson 100 von der Arbeitsplattform 60 aus Zugriff auf ein oder mehrere Sensoren, die an der Rühröffnung des Bioreaktors 2 ausgebildet sind.

Die Arbeitsplattform 60 weist eine begehbare Oberfläche auf, auf der die in Figur 4A gezeigte Bedienperson 100 steht bzw. stehen kann. Diese Oberfläche der Arbeitsplattform 60 ist als hohe Arbeitsplattform angeordnet und ausgebildet, ist bevorzugt zumindest etwa 65 cm vom Boden beabstandet, z.B. in einer Höhe von etwa 118 cm. Die begehbare Oberfläche der Arbeitsplattform ist unmittelbar benachbart zum Aufnahmebehälter 10 angeordnet und bevorzugt mindestens etwa 40 cm (z.B. etwa 45 cm) breit. Die begehbare Oberfläche erstreckt sich vom Aufnahmebehälter 10 weg (bevorzugt über eine Breite von etwa 45 cm) bis zu einem dem Aufnahmebehälter 10 abgewandten Plattformende. An diesem abgewandten Plattformende der Arbeitsplattform 60 bzw. der begehbaren Oberfläche der Arbeitsplattform 60 ist ein Geländer 62 ausgebildet, das die Bedienperson 100 vor einem Herunterfallen von der begehbaren Oberfläche schützt.

**Figur 4B** zeigt in einer Seitenansicht eine Vorrichtung 3 zum Aufnehmen eines Einwegbeutels mit einer (bevorzugt niedrigen) Arbeitsplattform 60. Die Vorrichtung 3 kann als Bioreaktor und/oder Bioreaktorsystem zum Aufnehmen eines Einwegbeutels ausgebildet sein.

Die niedrige Arbeitsplattform 60 der Vorrichtung 3 ist ähnlich wie diejenige der in Figur 2 gezeigten Vorrichtung 2 ausgebildet. Die niedrige Arbeitsplattform 60 der Vorrichtung 3 ist bevorzugt jedoch lediglich etwa 30 cm bis etwa 64 cm oberhalb des Bodens angeordnet und ist bevorzugt geländerlos ausgebildet.

In den Figuren nicht gezeigt ist eine Verdunklung, mit der die obere Rühröffnung der Vorrichtungen 1, 2 oder 3 verdunkelt werden kann. Die Verdunklung dient (bevorzugt zusammen mit der Türverdunklung und/oder den beiden Bodenfensterverdunklungen) zur Kontrolle und/oder Steuerung des Lichteinfalls in den Behälterinnenraum.

**Figur 5** zeigt in einer perspektivischen Darstellung einen zweiteiligen konischen Entleerungsanschluss 45. Der Entleerungsanschluss 45 ist dazu ausgebildet und vorgesehen, um einen Standardentleerungsanschluss eines Einwegbeutels 44 gelegt zu werden, um dem Entleerungsanschluss des Einwegbeutels 44 eine konische Außenfläche zu geben.

Der Entleerungsanschluss 45 ist bevorzugt als eigenständiges Bauteil ausgebildet und kann als Bauteil eines Bioreaktorsystems ausgebildet sein, wie z.B. der Vorrichtung 1, 2 oder 3. Der Entleerungsanschluss 45 ist bevorzugt zweiteilig ausgebildet und weist eine erste Teilschale 45A auf, sowie eine zweite Teilschale 45B. Die beiden Teilschalen 45A und 45B sind im Wesentlichen gleich groß ausgebildet und sind dazu ausgebildet und vorgesehen, zusammengesetzt zu werden, um den zusammengesetzten Entleerungsanschluss 45 zu bilden.

Jede der beiden Teilschalen 45A und 45B ist bevorzugt als Halbkonus ausgebildet, mit einer sich verjüngenden, im Wesentlichen konusförmigen Außenseite und/oder einer im Wesentlichen gerade ausgebildeten Schnittflächeninnenseite, in der eine Aufnahme 48 ausgebildet ist. Die Aufnahme 48 ist im Wesentlichen in Form eines Hohlzylinders ausgebildet und dient zur Aufnahme eines Standardenleerungsanschlusses eines Einwegbeutels. Die Schnittflächeninnenseiten der beiden Teilschalen bzw. Halbschalen 45A und 45B sind dazu ausgebildet und vorgesehen, flächig aufeinander gesetzt zu werden. So zusammengesetzt wird der (bevorzugt im Wesentlichen konusförmige) Entleerungsanschluss 45 bereitgestellt.

Die erste Teilschale 45A weist ein oder mehrere (z.B. zwei) Ausrichtungsstifte 46A auf, die an der Schnittflächeninnenseite ausgebildet sind und etwa im Lot von der Schnittflächeninnenseite wegstehen. Die ein oder mehrere (z.B. zwei) Ausrichtungsstifte 46A sind dazu ausgebildet und vorgesehen, in ein oder mehrere (z.B. zwei) komplementäre Ausrichtungsöffnungen 46B der zweiten Teilschale 45B zumindest teilweise eingeführt zu werden und dabei die beiden Teilschalen korrekt zueinander auszurichten. Zusätzlich sind an den Schnittflächeninnenseiten bevorzugt ein oder mehrere Magnete 47A und 47B angeordnet, die sich gegenseitig anziehen und/oder die beiden Teilschalen 45A und 45B zusammenziehen bzw. zusammenhalten. Die beiden Teilschalen können in einer alternativen Ausführungsform einen oder mehrere Scharnier(e) bzw. Gelenk(e) aufweisen, mit denen sie sicher in eine Sollposition aufeinander geklappt werden können.

Der in Figur 5 gezeigte Entleerungsanschluss 45 weist bevorzugt eine im Wesentlichen konusförmige, zylindersymmetrische Außenfläche auf. Alternativ dazu kann die Außenfläche des Entleerungsanschlusses auch im Wesentlichen halbkugelförmig und/oder in Form eines Halbellipsoids ausgebildet sein. Entscheidend bei der Außenform ist lediglich, dass sich der Entleerungsanschluss in seiner zusammengesetzten Form nach einer Richtung hin (vorzugsweise zylindersymmetrisch) verjüngt, und zwar in einer Richtung, die in Betriebsposition vom Inneren des Einwegbeutels weg weist.

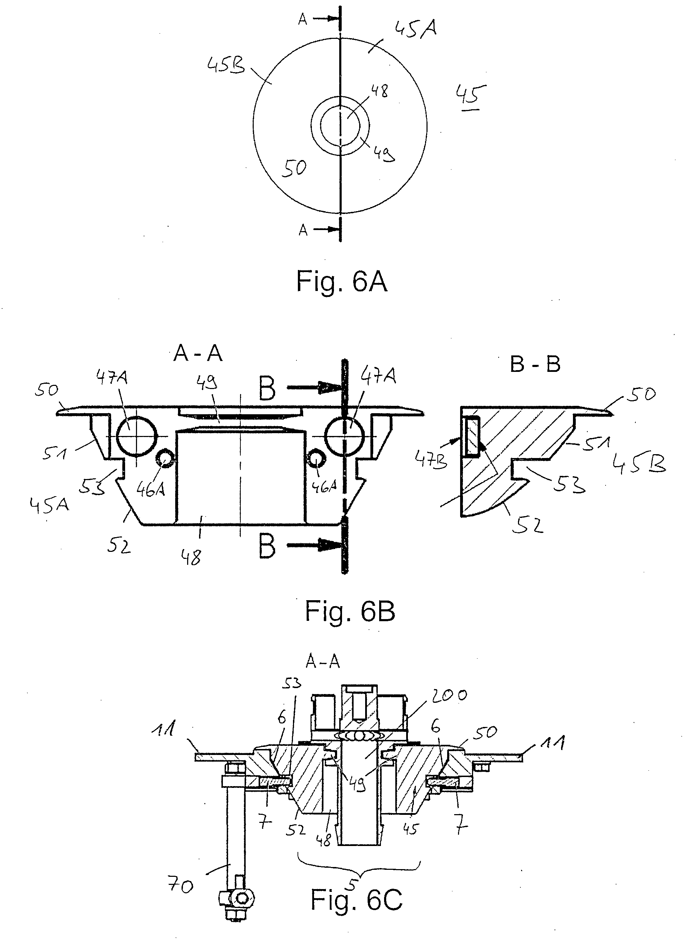

**Figur 6A** zeigt in einer Draufsicht den in Figur 5 gezeigten zweiteiligen konischen Entleerungsanschluss 45 in zusammengesetzter Form. Dabei ist deutlich die mittig angeordnete Aufnahme 48 gezeigt, und zwar in Achsrichtung entlang der Achse des Hohlzylinders, als der die Aufnahme 48 ausgebildet ist. In der gezeigten Draufsicht ist der Entleerungsanschluss 45 im Wesentlichen kreisförmig ausgebildet, wobei in der Kreismitte die Aufnahme 48 ausgebildet ist.

**Figur 6B** zeigt einen Querschnitt durch den in Figur 5 gezeigten zweiteiligen konischen Entleerungsanschluss 45. Links ist in dem Querschnitt lediglich die erste Teilschale 45A gezeigt, während rechts ein Querschnitt durch die zweite Teilschale 45B gezeigt ist, und zwar senkrecht zur Ebene der Schnittflächeninnenseite.

Der Entleerungsanschluss 45 ist bevorzugt im Wesentlichen zylindersymmetrisch ausgebildet bezüglich einer Mittelachse, die mit der Zylinderachse der Aufnahme 48 zusammenfällt. Der Entleerungsanschluss 45 weist am oberen Ende einen Flansch bzw. eine Kreisscheibe 50 auf, die von oben in Figur 6A gezeigt ist. Unterhalb des Flansches bzw. der Kreisscheibe 50 verjüngt sich die Außenfläche des Entleerungsanschlusses 45 entlang einer ersten Rutschfläche 51 und einer zweiten Rutschfläche 52, zwischen denen eine Umlaufnut 53 in der Außenfläche ausgebildet ist. Dabei ist die erste Rutschfläche 51 unterhalb der Kreisscheibe 50 ausgebildet, die Umlaufnut 53 unterhalb der ersten Rutschfläche 51, und die zweite Rutschfläche 52 unterhalb der Umlaufnut 53. Alternativ kann die Umlaufnut 53 auch unterhalb einer langen Rutschfläche angeordnet sein.

**Figur 6C** zeigt in einem Querschnitt den zweiteiligen konischen Entleerungsanschluss 45 in einer Betriebsposition, in der er um einen Standardentleerungsanschluss 200 gelegt ist. Der Entleerungsanschluss 45 ist mit seinen beiden Halbschalen so im Wesentlichen um den Standardentleerungsanschluss 200 eines handelsüblichen Einwegbeutels 44 gelegt bzw. angeordnet, dass der Einwegbeutel 44 nun den konusförmigen Entleerungsanschluss 45 als seinen Entleerungsanschluss aufweist, insbesondere genau dessen sich nach unten verjüngenden Außenmaße.

Dabei ist der Standardentleerungsanschluss 200 in der hohlzylinderförmigen Aufnahme 48 angeordnet, und zwar so, dass der im Wesentlichen zylinderfömige Standardentleerungsanschluss 200 mittels eines Umlaufvorsprungs 49 in der Aufnahme 48 gehalten wird. Der Umlaufvorsprung 49 ist als Teil des Entleerungsanschlusses 45 in der Aufnahme 48 ausgebildet, und zwar an jeder der beiden Teilschalen 45A und 45B. Der Umlaufvorsprung 49 weist in etwa senkrecht ins Innere des Hohlzylinders, also senkrecht zur Zylindersymmetrieachse des Entleerungsanschlusses 45, und dient zum Eingriff in eine entsprechende Vertiefung im Standardentleerungsanschluss 200, wie in Fig. 6C gezeigt. Die Vertiefung im Standardentleerungsanschluss 200 kann dabei üblicherweise zum Anschließen eines Schlauchs o.Ä. ausgebildet sein.

Durch die ein oder mehrere Magnete 47A und 47B und/oder die ein oder mehrere Ausrichtungsstifte 46A in den Ausrichtungsöffnungen 46B werden die beiden Teilschalen 45A, 45B fest und stabil um den Standardentleerungsanschluss 200 des Einwegbeutels gehalten bzw. angeordnet. Alternativ oder zusätzlich können die Teilschalen auch mittels eines Schnappverschlusses oder anderer Fixiermittel um den Standardentleerungsanschluss 200 des Einwegbeutels angeordnet werden. Der so mit dem Entleerungsanschluss 45 versehene Einwegbeutel 44 kann in eine der Vorrichtungen 1, 2 oder 3 eingebracht werden, die z.B. in den Figuren 1 bis 4 gezeigt sind. In diesen Vorrichtungen weist der Aufnahmebehälter 10 eine Bodenfläche 11 auf, in der eine sich nach unten verjüngende Auslassöffnung 5 ausgebildet ist, wie bereits in Fig. 3 klein und als vergrößerter Ausschnitt in Fig. 6C gezeigt.

Die Auslassöffnung 5 ist bevorzugt im Wesentlichen zylindersymmetrisch um eine vertikale Zylinderachse ausgebildet und weist sich nach unten verjüngende Innenflächen auf, die als Sitzflächen 6 für die Außenflächen des Entleerungsanschlusses 45 ausgebildet ist. Die Sitzflächen 6 sind im Wesentlichen komplementär zu den Außenmaßen des Entleerungsanschlusses 45 ausgebildet und/oder auf die Verjüngung der ersten und zweiten Rutschfläche 51 und 52 im Wesentlichen abgestimmt und an diese angepasst. Insbesondere sind im Querschnitt die Steigungen aufeinander abgestimmt. In anderen Worten ist die Abnahme des Innenradius entlang der Symmetrieachse der Auslassöffnung 5 nach unten gleich der Abnahme des Außenradius entlang der Symmetrieachse des Entleerungsanschlusses 45 nach unten. Die Sitzflächen 6 weisen somit im Wesentlichen die Form eines sich nach unten verjüngenden Konus auf.

Beim Einbringen des Einwegbeutels 44 in die Vorrichtung 1, 2 bzw. 3 rutscht bzw. gleitet der Entleerungsanschluss 45 ohne viel Zutun einer Bedienperson, also praktisch von alleine, in die Auslassöffnung 5 in der Bodenfläche 11 des Aufnahmebehälters. Der Entleerungsanschluss 45 kann dabei an einem Schlauch- oder Schnurende in die Aufnahmeöffnung 5 geführt werden. Dabei rutscht der Entleerungsanschluss 45 mitsamt dem umschlossenen Standardentleerungsanschluss 200 so weit in die Auslassöffnung 5, bis der Flansch bzw. die Kreisscheibe 50 auf der Auslassöffnung 5 aufliegt. Die Kreisscheibe 5 weist einen Außendurchmesser auf, der größer als der Innendurchmesser der Auslassöffnung 5 ausgebildet ist. Deswegen liegt der Flansch bzw. die Kreisscheibe 50 in der in Figur 6C gezeigten Betriebsposition flach auf der Bodenfläche 11 auf, zentriert um die Symmetrieachse der Auslassöffnung 5. Bis auf die Kreisscheibe 11 ist der Entleerungsanschluss 45 in der Betriebsposition vollständig in der Auslassöffnung 5 angeordnet und durchdringt diese sogar. Insbesondere liegen die erste Rutschfläche 51 und die zweite Rutschfläche 52 bündig auf der Sitzfläche 6 auf. Weiterhin fällt in der in Fig. 6C gezeigten Betriebsposition die Symmetrieachse des Entleerungsanschlusses 45 in etwa mit der Symmetrieachse der Auslassöffnung 5 zusammen.

In der Auslassöffnung 5 ist zumindest ein Fixierelement 7 bevorzugt in Form eines Bolzens angeordnet, das in einer Schließposition zumindest teilweise in die Umlaufnut 53 des Entleerungsanschlusses 45 eingreift und diesen in der Auslassöffnung 5 fixiert. Das Fixierelement 7 ist dabei bevorzugt etwa im Lot zur Mittelachse (also der Symmetrieachse) der Auslassöffnung 5 beweglich zwischen einer Schließposition und einer Offenposition. Durch das Fixierelement 7 wird eine Verriegelung der Auslassöffnung 5 bereitgestellt.

In Figur 6C ist die Schließposition der Verriegelung, also des Fixierelements 7, gezeigt, in welcher der Entleerungsanschluss 45 mittels des Fixierelements 7 in der Auslassöffnung 5 in der Betriebsposition fixiert ist.

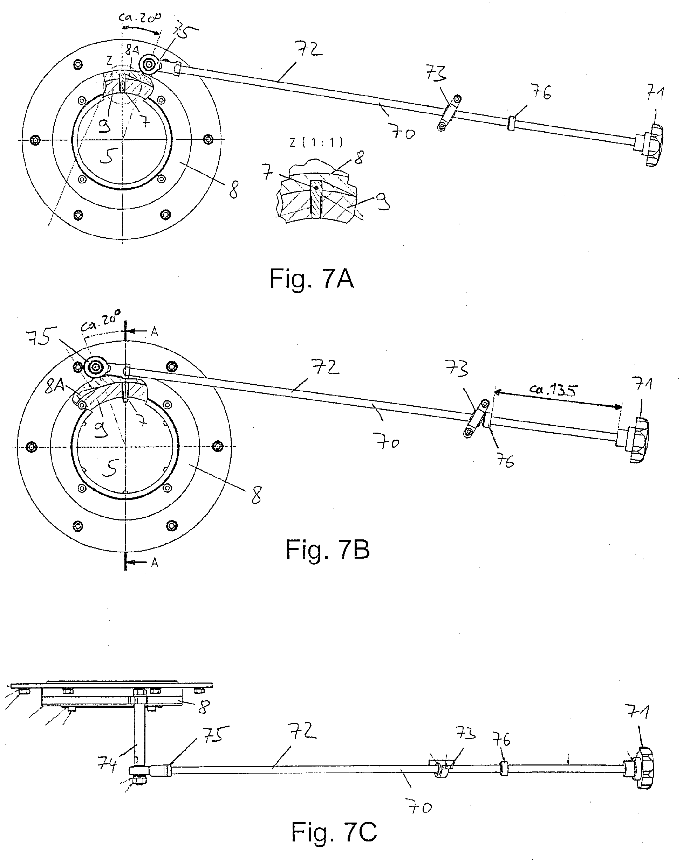

Das Fixierelement 7 kann mittels einer Fernbedienung 70 geöffnet und geschlossen werden. Diese Fernbedienung 70 ist als mechanische Fernbedienung ausgebildet, die in Figur 6C lediglich teilweise gezeigt ist, aber genauer in den Figuren 7A-7C.

**Figur 7A** zeigt in einer Bodenansicht (also in einer Ansicht von unten vertikal nach oben) die sich nach unten verjüngenden Auslassöffnung 5 mit geöffnetem Fixierelement 7 sowie mit der zugehörigen mechanischen Fernbedienung 70. Die Fernbedienung 70 ist mittels eines Griffs 71 betätigbar, an welchem eine Horizontalstange 72 angeordnet ist bzw. werden kann, die in einer Öse 73 bis zu einem Anschlag 76 in eine im Wesentlichen horizontale Richtung bewegbar ist.

**Figur 7C** zeigt die Fernbedienung 70 in einer Seitenansicht. In der in den Figuren 7A und 7C gezeigten Offenposition der Verriegelung ist der Griff 71 maximal von der Auslassöffnung 5 weg bewegt. Der Griff 71 ist an einem Ende der Horizontalstange 72 angeordnet, der von der Auslassöffnung 5 abgewandt ist. Ein gegenüberliegendes Ende der Horizontalstange 72 ist als Gelenk 75 ausgebildet. In diesem Gelenk 75 ist eine Vertikalstange 74 angeordnet, die an einem Drehring 8 befestigt ist.

Die Vertikalstange 74 ist bevorzugt im Wesentlichen vertikal ausgerichtet und steht somit in etwa senkrecht zur Horizontalstange 72, die bevorzugt im Wesentlichen horizontal angeordnet ist. Die Vertikalstange 74 kann kürzer als die Horizontalstange 72 ausgebildet sein. Auf die Vertikalstange 74 kann in einer alternativen Ausführungsform verzichtet werden, insbesondere bei anders zugänglichen Bioreaktorsystemen.

Der Drehring 8 ist um die Auslassöffnung 5 drehbar gelagert, genauer um einen Fixring 9 (siehe Figur 7A). Der Drehring 8 ist um die vertikale Mittelachse (also die Symmetrieachse) der Auslassöffnung 5 drehbar, und zwar zwischen der Schließposition und der Offenposition. Letztere ist in den Figuren 7A und 7C gezeigt. Der Fixring 9 ist um zumindest einen Teil der Auslassöffnung 5 herum angeordnet, in seiner Position unbeweglich und dient als innerer Drehanker, um den der außen angeordnete Drehring 8 drehbar gelagert ist.

Alternativ oder zusätzlich zu dem Drehring kann die Vorrichtung einen Schieber aufweisen, mit dem der Entleerungsanschluss 45 in oder an der Auslassöffnung 5 verriegelt werden kann.

In der Offenposition ist der Drehring 8 relativ zum Fixring 9 so ausgerichtet, dass eine Aussparung 8A in der Innenfläche des Drehrings 8 genau auf Höhe des Fixierelements 7 angeordnet ist. Das Fixierelement 7 weist eine Federung auf, die das Fixierelement 7 von der Mittelachse der Auslassöffnung 5 radial weg nach außen drückt, und zwar in der Offenposition in die Aussparung 8A im Drehring 8 hinein. So nach außen gedrückt ragt das Fixierelement 7 nicht mehr ins Innere der Auslassöffnung 5 hinein, sondern schließt im Wesentlichen bündig mit den Sitzflächen 6 der Auslassöffnung 5 ab und gibt dabei im Wesentlichen die gesamte Auslassöffnung 5 frei.

Die genaue Stellung des Drehrings 8 relativ zum Fixring 9, und insbesondere das Eingreifen des Fixierelements 7 in die Aussparung 8A, ist in Figur 7A noch einmal in einem vergrößerten Ausschnitt gezeigt. In der Offenposition kann der konusförmige Entleerungsanschluss 45 eines Einwegbeutels ungehindert in die Auslassöffnung 45 hereinrutschen.

Die Schließposition, in der das Fixierelement 7 in Richtung zur Mittelachse ins Innere der Auslassöffnung 5 hervorsteht, ist in **Figur 7B** gezeigt. Um von der Offenposition in die Schließposition zu gelangen, wird der Griff 71 der Fernbedienung 70 in Richtung auf die Auslassöffnung zu bewegt. Dabei wird die Horizontalstange 72 durch die ortsfeste Öse 73 bewegt, bis der Anschlag 76 an der Öse 73 anschlägt. Der Anschlag 76 ist größer als die Ösenöffnung der Öse 73 ausgebildet.

Bei dieser Schließbewegung wird das Gelenk 75 und somit auch die Vertikalstange 74 in Richtung vom Griff 71 weg bewegt. Hierbei dreht sich der Drehring 8 um den Fixring 9. Dabei wird die Aussparung 8A relativ zum Fixierelement 7 bewegt, nämlich von diesem weg. In der Schließposition liegt der Drehring 8 mit seiner Innenseite unmittelbar an der Außenseiten des Fixrings 9 an und drückt bzw. beaufschlägt dabei das Fixierelement 7 ins Innere der Auslassöffnung 5. In der Schließposition ist die Aussparung 8A somit relativ zum Fixierelement 7 bevorzugt versetzt angeordnet.

Die Drehung des Drehrings 8 von der Offenposition in die Schließposition kann über einen Drehwinkel von etwa 20° bis etwa 60° erfolgen, in dem in den Figuren gezeigten Ausführungsbeispiel über einen Drehwinkel von etwa 40°.

Wird der Griff 71 wieder von der Auslassöffnung 5 weg bewegt, dreht sich der Drehring 8 zurück bis die Aussparung 8A wieder auf Höhe des Fixierelements 7 angeordnet ist, das in die Aussparung 8A hereinspring und die weitere Drehbewegung blockiert. Das Fixierelement 7 dient somit zugleich als Begrenzung der Öffnungsbewegung, während der um die Horizontalstange 72 ausgebildete Anschlag 73 die Schließbewegung begrenzt.

Die Fernbedienung 70 erleichtert ein Fixieren des Entleerungsanschlusses 45 in der Auslassöffnung 5 an der schwer zugänglichen Bodenfläche 11 des Aufnahmebehälters 10.

Anstelle des Entleerungsanschlusses 45, der als separates Bauteil um einen Standardentleerungsanschluss gelegt wird, kann ein Einwegbeutel mit einem bereits integrierten (bevorzugt konusförmigen) Entleerungsanschluss zum Einbringen in das Bioreaktorsystem verwendet werden.

In einer alternativen (nicht gezeigten) Ausführungsform kann der Entleerungsanschluss auch aus mehr als lediglich zwei Bauteilen aufgebaut sein, und/oder anstatt (oder zusätzlich) zu den Magneten mechanische Fixiervorrichtungen wie etwa Rasthaken etc. vorgesehen sein.

Der Verriegelungsmechanismus der Auslassöffnung kann nicht lediglich zum Verriegeln des Entleerungsanschlusses verwendet werden, sondern auch zur Fixierung eines beliebigen anderen Anschlusses des Einwegbeutels in einer anderen Beutellage.

Alternativ zur dargestellten Verriegelung mit dem Drehring kann der Entleerungsanschluss auch über einen in die Umlaufnut eingreifenden Schiebemechanismus fixiert werden.

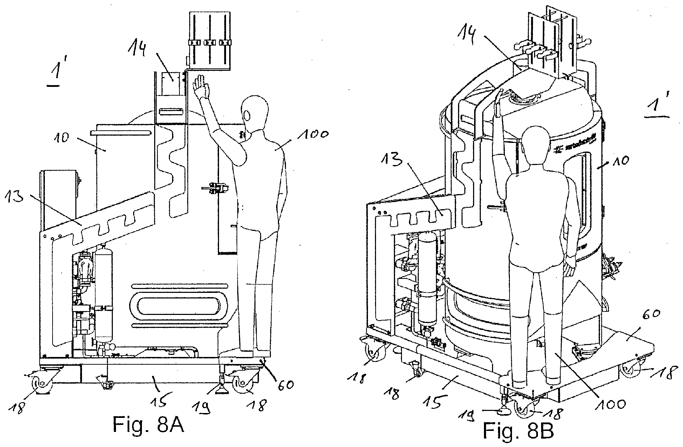

**Figuren 8A und 8B** zeigen in einer Seitenansicht und in einer perspektivischen Darstellung eine Vorrichtung 1' zum (zumindest teilweisen) Aufnehmen eines Einwegbeutels mit einer Arbeitsplattform 60. Die Vorrichtung 1' kann insbesondere als Bioreaktor und/oder Bioreaktorsystem zum Aufnehmen eines Einwegbeutels ausgebildet sein.

An einer Seite des Bioreaktors 1' ist die Arbeitsplattform 60 ausgebildet, auf der eine Bedienperson 100 steht und die Rührvorrichtung 14 bedient. Weiterhin hat die Bedienperson 100 von der Arbeitsplattform 60 aus Zugriff auf ein oder mehrere Sensoren, die an der Rühröffnung des Bioreaktors 1' ausgebildet sind.

Die Arbeitsplattform 60 weist eine begehbare Oberfläche auf, auf der die in den Figuren 8A und 8B gezeigte Bedienperson 100 steht bzw. stehen kann. Diese Oberfläche der Arbeitsplattform 60 ist als Arbeitsplattform angeordnet und ausgebildet.

Die begehbare Oberfläche der Arbeitsplattform ist benachbart zum Aufnahmebehälter 10 angeordnet und bevorzugt zumindest an einer Ecke der Vorrichtung 1' zumindest etwa 40 cm breit. Die begehbare Oberfläche erstreckt sich vom Aufnahmebehälter 10 weg bis zu einem dem Aufnahmebehälter 10 abgewandten Plattformende.

Die Arbeitsplattform 60 ist als eine Trittfläche ausgebildet, die die Bedienperson 100 betreten kann, um leichter an die Rührvorrichtung 14 und/oder den zugehörigen Motor gelangen zu können. Im gezeigten Ausführungsbeispiel ist die Arbeitsplattform 60 als begehbare Oberfläche ausgebildet, die z.B. unmittelbar auf der Auffangwanne 15 aufliegt und/oder an der Auffangwanne 15 befestigt ist, insbesondere auf bzw. an einer Oberkante der Auffangwanne 15. Dadurch kann die Arbeitsplattform 60 besonders unaufwendig, kostengünstig und/oder Platz sparend implementiert werden.

Die Auffangwanne 15 ist hierbei so ausgebildet, dass der Aufnahmebehälter 10 in der Auffangwanne 15 steht. Weiterhin sind die Rollen 18 und/oder die Fixierfüße 19 der Vorrichtung 1' so ausgebildet, dass sie in die Auffangwanne 15 eingelassen und/oder integriert sind. Die Auffangwanne 15 ist im Vergleich zu den in den Figuren 4A und 4B gezeigten Ausführungsformen sozusagen "tiefer gelegt", wodurch die Gesamthöhe der Vorrichtung 1' reduziert werden kann. Dies kann dadurch erreicht werden, dass die Rollen 18 und/oder die Fixierfüße 19 zumindest teilweise an einer Oberkante der Auffangwanne 15 ansetzen und/oder an dieser Oberkante befestigt sind, und somit entweder keinen oder lediglich einen reduzierten Platzbedarf unterhalb der Auffangwanne 15 benötigen.

So können z.B. vier der Rollen 18 an Ecken der Auffangwanne 15 angeordnet sein, die lateral vom Aufnahmebehälter 10 abstehen und deswegen kein Auffangvolumen aufweisen müssen. An diesen Positionen kann die Auffangwanne 15 somit ausreichend beabstandet vom Boden angeordnet sein, was einen hinreichenden Freiraum für die Rollen 18 bereitstellt.

Unterhalb des Aufnahmebehälters 10 kann die Auffangwanne 15 ein Auffangvolumen aufweisen, das bis nahe zum Boden reicht. Mit anderen Worten kann die Auffangwanne 15 unterhalb des Aufnahmebehälters 10 maximal etwa 5 cm vom Boden beabstandet angeordnet sein, bevorzugt maximal etwa 3 cm, besonders bevorzugt maximal etwa 1 cm beabstandet.

Hierbei kann die Bauhöhe der Vorrichtung 1' reduziert werden, und gleichzeitig eine Erreichbarkeit der Rührvorrichtung 14 und/oder des zugehörigen Motors für die Bedienperson 100 verbessert werden.

Die Ausbildung der Arbeitsplattform 60 als relativ niedriges Trittbrett über der tiefer gelegten Auffangwanne 15 ermöglicht es zudem, auf ein Geländer 62 der Arbeitsplattform 60 zu verzichten, wie es z.B. bei der erhöhten Arbeitsplattform 60 in Fig. 4A gezeigt ist. Dadurch wird ein Bauteilaufwand bei der Herstellung der Vorrichtung 1' reduziert.

### Bezugszeichenliste

- 1: Vorrichtung
- 1': Vorrichtung
- 2: Vorrichtung
- 3: Vorrichtung
- 5: Auslassöffnung
- 6: Sitzfläche
- 7: Fixierelement
- 8: Drehring
- 8A: Aussparung
- 9: Fixring
- 10: Aufnahmebehälter
- 11: Bodenfläche
- 12: Bodenfenster
- 13: Kabelführung
- 13A: Umschwenkpunkt
- 13B: Unterkabelführung
- 13D: Steckklappen
- 13E: Oberkabelführung
- 14: Rührvorrichtung
- 14A: Rührelementaufnahme
- 14B: Querelement
- 14C: Trägerelement
- 14D: Motor
- 14E: Abstützbereich
- 15: Auffangwanne
- 16: Behälterwand
- 17: Systemschiene
- 18: Rollen
- 19: Fixierfüße
- 20: Temperierhohlwand
- 25: Isolierhohlwand
- 30: Einblatttür
- 32: Türsichtfenster
- 33: Türverdunklung
- 34: Türscharnier
- 35: Türgriff
- 40: Füllstandhöhe
- 42: biologisches Medium
- 44: Einwegbeutel
- 45: Entleerungsanschluss
- 45A: erste Teilschale
- 45B: zweite Teilschale
- 46A: Ausrichtungsstift
- 46B: Ausrichtungsöffnung
- 47A: Magnet
- 47B: Magnet
- 48: Aufnahme
- 49: Umlaufvorsprung
- 50: Kreisscheibe
- 51: erste Rutschfläche
- 52: zweite Rutschfläche
- 53: Umlaufnut
- 60: Arbeitsplattform
- 61: Leiter
- 62: Geländer
- 70: Fernbedienung
- 71: Griff
- 72: Horizontalstange
- 73: Öse
- 74: Vertikalstange
- 75: Gelenk
- 76: Anschlag
- 100: Bedienperson
- 200: Standardentleerungsanschluss

## Patentansprüche

1. Bioreaktorsystem (1; 2; 3) zum Aufnehmen eines Einwegbeutels (44),
mit einem Aufnahmebehälter (10) zum Aufnehmen des Einwegbeutels (44), wobei
eine Bodenfläche (11) des Aufnahmebehälters (10) zum Stützen des Einwegbeutels (44) im Aufnahmebehälter (10) ausgebildet ist und
in der Bodenfläche (11) eine sich im Bezugssystem der Erde nach unten verjüngende Auslassöffnung (5) zur Aufnahme eines zur Auslassöffnung (5) komplementären Entleerungsanschlusses (45) des Einwegbeutels (44) angeordnet ist.

2. Bioreaktorsystem nach Anspruch 1, wobei sich die Auslassöffnung (5) zumindest teilweise nach unten hin im Wesentlichen konusförmig und/oder im Wesentlichen halbkugelförmig und/oder im Wesentlichen in Form eines Halbellipsoids verjüngt.

3. Bioreaktorsystem nach einem der Ansprüche 1 oder 2, mit einem Fixierelement (7) zum Fixieren des komplementären Entleerungsanschlusses (45) des Einwegbeutels (44) in der Auslassöffnung (5).

4. Bioreaktorsystem nach Anspruch 3, mit einer Fernbedienung (70) zum Öffnen und/oder Schließen des Fixierelements (7).

5. Bioreaktorsystem nach einem der vorangegangenen Ansprüche, wobei der zur Auslassöffnung (5) komplementäre Entleerungsanschluss (45) als eigenständiges Bauteil des Bioreaktorsystems (1; 2; 3) ausgebildet ist, das an einem im Einwegbeutel (44) integrierten Standardentleerungsanschluss (200) so befestigbar ist, dass sich der komplementäre Entleerungsanschluss (45) in einer Richtung verjüngt, die vom Beutelinneren des Einwegbeutels (44) weg weist.

6. Bioreaktorsystem nach Anspruch 5, wobei der komplementäre Entleerungsanschluss (45) als mehrteiliges Bauteil ausgebildet ist.

7. Bioreaktorsystem (1; 2; 3) zum Aufnehmen eines Einwegbeutels (44) nach einem der vorangegangenen Ansprüche, mit einer im Wesentlichen an eine Außenwand des Aufnahmebehälters (10) angrenzend angeordneten Arbeitsplattform (60),
wobei eine von einer Bedienperson (100) begehbare Oberfläche der Arbeitsplattform (60) etwa 80 cm bis etwa 140 cm unterhalb einer Oberkante des Aufnahmebehälters (10) angeordnet ist.

8. Bioreaktorsystem nach Anspruch 7, wobei die Arbeitsplattform (60) zumindest teilweise ausziehbar und/oder ausklappbar ausgebildet ist.

9. Bioreaktorsystem nach einem der vorangegangenen Ansprüche, mit einem Aufnahmebehälter (10) zum Aufnehmen des Einwegbeutels (44) und einer unterhalb des Aufnahmebehälters (10) angeordneten Auffangwanne (15) zum Auffangen eines aus dem Aufnahmebehälter (10) austretenden Fluids.

10. Bioreaktorsystem nach einem der vorangegangenen Ansprüche, mit einer Systemschiene (17) zum Anbringen von zumindest einer Zusatzvorrichtung.

11. Bioreaktorsystem (1; 2; 3) zum Aufnehmen eines Einwegbeutels (44) nach einem der vorangegangenen Ansprüche, mit einer Kabelführung (13) an einer Außenwand des Aufnahmebehälters (10), wobei die Kabelführung (13) bis zu einer Oberkante des Aufnahmebehälters (10) ausgebildet ist.

12. Bioreaktorsystem nach Anspruch 11, wobei die Kabelführung (13) zumindest teilweise mäanderförmig ausgebildet ist;
und/oder
wobei die Kabelführung (13) von der Oberkante des Aufnahmebehälters (10) an dessen Außenwand entlang im Wesentlichen vertikal nach unten verläuft bis zu einem Umschwenkpunkt (13A), an welchem die Kabelführung (13) von der Außenwand des Aufnahmebehälters (10) weg zu einem Außenbereich des Bioreaktorsystems (1; 2; 3) verläuft.

13. Bioreaktorsystem nach einem der vorangegangenen Ansprüche, mit einer Verdunklungsvorrichtung (33) für zumindest ein Sichtfenster (32) und/oder einer Verdunklungsabdeckung zum Verdunkeln einer Öffnung an einer Oberkante des Aufnahmebehälters (10).

14. Verfahren zum Aufnehmen eines Einwegbeutels (44) in ein Bioreaktorsystem (1; 2; 3), mit den Schritten:
- Bereitstellen eines Bioreaktorsystems (1; 2; 3) mit einem Aufnahmebehälter (10), in dessen Bodenfläche (11) eine sich im Bezugssystem der Erde nach unten verjüngende Auslassöffnung (5) ausgebildet ist;
- Bereitstellen eines Einwegbeutels (44) mit einem Standardentleerungsanschluss (200);
- Anbringen eines zur Auslassöffnung (5) komplementären Entleerungsanschlusses (45) an den Standardentleerungsanschluss (200) des Einwegbeutels (44);
- Einbringen des Einwegbeutels (44) in den Aufnahmebehälter (10) des Bioreaktorsystems (1; 2; 3), wobei der komplementäre Entleerungsanschluss (45) in der Auslassöffnung (5) angeordnet wird.

15. Verfahren nach Anspruch 14, wobei ein Schlauch durch die Auslassöffnung (5) geführt wird, dessen eines Schlauchende am komplementären Entleerungsanschluss (45) angebracht wird, wobei der komplementäre Entleerungsanschluss (45) mit Hilfe des Schlauchs in die sich nach unten verjüngende Auslassöffnung (5) geführt wird.

## Claims

1. A bioreactor system (1; 2; 3) for accommodating a disposable bag (44) with an accommodating container (10) for accommodating the disposable bag (44),
wherein
a bottom surface (11) of the accommodating container (10) is designed to support the disposable bag (44) in the accommodating container (10), and
an outlet opening (5) in the bottom surface (11) that, in the reference system of the earth, tapers downward for accommodating a drain connection (45), complementary with the outlet opening (5), of the disposable bag (44).

2. The bioreactor system according to claim 1, wherein the outlet opening (5) tapers at least partially downward basically conically, and/or basically hemispherically, and/or basically in the form of a half ellipsoid.

3. The bioreactor system according to one of claims 1 or 2 with a fixing element (7) for fixing the complementary drain connection (45) of the disposable bag (44) in the outlet opening (5).

4. The bioreactor system according to claim 3, with a remote control (70) for opening and/or closing the fixing element (7).

5. The bioreactor system according to one of the preceding claims, wherein the drain connection (45) complementary to the outlet opening (5) is designed as an independent component of the bioreactor system (1; 2; 3) which can be fastened to a standard drain connection (200) integrated in the disposable bag (44) such that the complementary drain connection (45) tapers in a direction facing away from the interior of the disposable bag (44).

6. The bioreactor system according to claim 5, wherein the complementary drain connection (45) is designed as a multipart component.

7. The bioreactor system (1; 2; 3) for accommodating a disposable bag (44) according to one of the preceding claims, with a work platform (60) arranged substantially adjacent to an outer wall of the accommodating container (10),
wherein a surface of the work platform (60) accessible to an operator (100) is arranged about 80 cm to about 140 cm below a top edge of the accommodating container (10).

8. The bioreactor according to claim 7, wherein the work platform (60) is designed at least partially removable and/or foldable.

9. The bioreactor system according to one of the preceding claims with an accommodating container (10) for accommodating the disposable bag (44) and a drain pan (15) arranged below the accommodating container (10) for capturing fluid leaving the accommodating container (10).

10. The bioreactor system according to one of the preceding claims with a system rail (17) for attaching at least one additional device.

11. The bioreactor system (1; 2; 3) for accommodating a disposable bag (44) according to one of the preceding claims, with a cable run (13) on an outer wall of the accommodating container (10) wherein the cable run (13) is formed up to a top edge of the accommodating container (10).

12. The bioreactor according to claim 11, wherein the cable run (13) is designed at least partially meandering;
and/or
wherein the cable run (13) runs downward from the top edge of the accommodating container (10) to its outer wall substantially vertically up to a deflection point (13A) at which the cable run (13) runs from the outer wall of the accommodating container (10) away to an exterior area of the bioreactor system (1; 2; 3).

13. The bioreactor system according to one of the preceding claims with a darkening device (33) for at least one inspection window (32), and/or a darkening cover for darkening an opening in a top edge of the accommodating container (10).

14. A method for accommodating a disposable bag (44) in a bioreactor system (1; 2; 3) having the steps:
- providing a bioreactor system (1; 2; 3) having an accommodating container (10) in the bottom surface (11) of which an outlet opening (5) is formed that tapers downward in the reference system of the earth;
- providing a disposable bag (44) having a standard drain connection (200);
- attaching a drain connection (45) that is complementary with the outlet opening (5) to the standard drain connection (200) of the disposable bag (44);
- introducing the disposable bag (44) into the accommodating container (10) of the bioreactor system (1; 2; 3), wherein the complementary drain connection (45) is arranged in the outlet opening (5).

15. The method according to claim 14, wherein a hose is guided through the outlet opening (5), one hose end of which is attached to the complementary drain connection (45), wherein the complementary drain connection (45) is guided into the downward-tapering outlet opening (5) with the assistance of the hose.

## Revendications

1. Système de bioréacteur (1 ; 2 ; 3) destiné à recevoir un sac jetable (44), doté
d'une cuve collectrice (10) servant à recevoir le sac jetable (44),
dans lequel
une surface de fond (11) de la cuve collectrice (10) est conçue pour soutenir le sac jetable (44) dans la cuve collectrice (10) et
un orifice de sortie (5) se rétrécissant vers le bas dans le système de référence terrestre est disposé dans la surface de fond (11) pour recevoir un raccord d'évacuation (45) du sac jetable (44), complémentaire de l'orifice de sortie (5).

2. Système de bioréacteur selon la revendication 1, dans lequel l'orifice de sortie (5) se rétrécit au moins en partie vers le bas sous une forme sensiblement conique et/ou sous une forme sensiblement hémisphérique et/ou sous une forme sensiblement semi-ellipsoïdale.

3. Système de bioréacteur selon l'une des revendications 1 ou 2, doté d'un élément de fixation (7) pour attacher le raccord d'évacuation (45) complémentaire du sac jetable (44) dans l'orifice de sortie (5).

4. Système de bioréacteur selon la revendication 3, doté d'une télécommande (70) pour ouvrir et/ou fermer l'élément de fixation (7).

5. Système de bioréacteur selon l'une des revendications précédentes, dans lequel le raccord d'évacuation (45) complémentaire de l'orifice de sortie (5) est conçu comme un composant à part entière du système de bioréacteur (1 ; 2 ; 3), qui peut être fixé à un raccord d'évacuation standard (200) intégré au sac jetable (44), de sorte que le raccord d'évacuation complémentaire (45) se rétrécit dans une direction, qui s'éloigne de l'intérieur du sac jetable (44).

6. Système de bioréacteur selon la revendication 5, dans lequel le raccord d'évacuation complémentaire (45) est conçu comme un composant en plusieurs parties.

7. Système de bioréacteur (1 ; 2 ; 3) destiné à recevoir un sac jetable (44) selon l'une des revendications précédentes, doté d'une plate-forme de travail (60) disposée de manière sensiblement adjacente à une paroi extérieure de la cuve collectrice (10),
dans lequel une surface de la plateforme de travail (60) accessible par un opérateur (100) est disposée à une hauteur d'environ 80 cm jusqu'à environ 140 cm en dessous d'un bord supérieur de la cuve collectrice (10).

8. Système de bioréacteur selon la revendication 7, dans lequel la plateforme de travail (60) est conçue de manière à être au moins en partie extensible et/ou pliable.

9. Système de bioréacteur selon l'une des revendications précédentes, doté d'une cuve collectrice (10) servant à recevoir le sac jetable (44) et d'un bac de récupération (15) disposé en dessous de la cuve collectrice (10) pour recueillir un fluide s'écoulant de la cuve collectrice (10).

10. Système de bioréacteur selon l'une des revendications précédentes, doté d'un rail de système (17) servant à mettre en place au moins un dispositif auxiliaire.

11. Système de bioréacteur (1 ; 2 ; 3) destiné à recevoir un sac jetable (44) selon l'une des revendications précédentes, doté d'un chemin de câbles (13) sur une paroi extérieure de la cuve collectrice (10), dans lequel le chemin de câbles (13) est conçu jusqu'à un bord supérieur de la cuve collectrice (10).

12. Système de bioréacteur selon la revendication 11, dans lequel le chemin de câbles (13) est conçu au moins en partie sous forme de méandres ;
et/ou
dans lequel le chemin de câbles (13) s'étend du bord supérieur de la cuve collectrice (10) le long de sa paroi extérieure sensiblement à la verticale vers le bas jusqu'à un point de changement de direction (13A), au niveau duquel le chemin de câbles (13) s'éloigne de la paroi extérieure de la cuve collectrice (10) pour atteindre une zone extérieure du système de bioréacteur (1 ; 2 ; 3).

13. Système de bioréacteur selon l'une des revendications précédentes, doté d'un dispositif d'obscurcissement (33) d'au moins un hublot (32) et/ou un cache d'obscurcissement pour opacifier une ouverture sur une bord supérieur de la cuve collectrice (10).

14. Procédé de réception d'un sac jetable (44) dans un système de bioréacteur (1 ; 2 ; 3), présentant les étapes suivantes :
- la mise à disposition d'un système de bioréacteur (1 ; 2 ; 3) doté d'une cuve collectrice (10), dans la surface de fond (11) de laquelle est constitué un orifice de sortie (5) se rétrécissant vers le bas dans le système de référence terrestre ;
- la mise à disposition d'un sac jetable (44) doté d'un raccord d'évacuation standard (200) ;
- la pose d'un raccord d'évacuation (45) complémentaire de l'orifice de sortie (5) sur le raccord d'évacuation standard (200) du sac jetable (44) ;
- la mise en place du sac jetable (44) dans la cuve collectrice (10) du système de bioréacteur (1 ; 2 ; 3), dans lequel le raccord d'évacuation complémentaire (45) est disposé à l'intérieur de l'orifice de sortie (5).

15. Procédé selon la revendication 14, dans lequel un tuyau chemine à travers l'orifice de sortie (5), dont une extrémité de tuyau est posée sur le raccord d'évacuation complémentaire (45), dans lequel le raccord d'évacuation complémentaire (45) est guidé dans l'orifice de sortie (5) se rétrécissant vers le bas à l'aide du tuyau.
